# EUROPEAN PATENT APPLICATION

(11) **EP 4 029 508 A1**
(43) Date of publication of application: **20.07.2022**
(21) Application number: 21212650.2
(22) Date of filing: 09.10.2015
(51) Int. Cl.: A61K 31/7115, A61K 39/39, A61K 45/06, A61K 9/00, A61P 35/00, A61P 35/02, A61P 35/04, A61P 43/00, A61K 39/00, A61N 5/00, C07K 14/435

(54) **TREATMENT OF CANCER USING TLR9 AGONISTS AND CHECKPOINT INHIBITORS**

(30) Priority: 10.10.2014 US 201462062274 P; 15.09.2015 US 201562218934 P
(62) Divisional of application: 15848691.0
(71) Applicant: Idera Pharmaceuticals, Inc., Cambridge, MA 02319 (US)
(72) Inventor: WANG, Daqing, Bedford, 01730 (US); JIANG, Wayne, Waltham, 02452 (US); AGRAWAL, Sudhir, Shrewsbury, 01545 (US)
(74) Representative: Grund, Martin

(57) **Abstract**

The invention provides methods of inducing an immune response to cancer comprising coadministering to a cancer patient one or more TLR9 agonists and one or more checkpoint inhibitors. Preferably, the one or more TLR9 agonists are administered to the patient via intratumoral (i.t.) administration.

## Description

### RELATED APPLICATIONS

This application claims the benefit of U.S. Provisional Application No. 62/062,274, filed on October 10, 2014 and U.S. Provisional Application No. 62/218,934, filed on September 15, 2015. The entire teachings of the above application(s) are incorporated herein by reference.

### BACKGROUND OF THE INVENTION

### Field of the invention

The invention generally relates to the field of oncology, and more specifically the use of immunotherapy in the treatment of cancer.

### Summary of the related art

Toll-like receptors (TLRs) are present on many cells of the immune system and have been shown to be involved in the innate immune response (Hornung, V. et al, (2002) J. Immunol. 168:4531-4537). In vertebrates, this family consists of eleven proteins called TLR1 to TLR11 that are known to recognize pathogen associated molecular patterns from bacteria, fungi, parasites, and viruses (Poltorak, A. et al. (1998) Science 282:2085-2088; Underhill, D.M., et al. (1999) Nature 401:811-815; Hayashi, F. et. al (2001) Nature 410:1099-1103; Zhang, D. et al. (2004) Science 303:1522-1526; Meier, A. et al. (2003) Cell. Microbiol. 5:561-570; Campos, M.A. et al. (2001) J. Immunol. 167: 416-423; Hoebe, K. et al. (2003) Nature 424: 743-748; Lund, J. (2003) J. Exp. Med. 198:513-520; Heil, F. et al. (2004) Science 303:1526-1529; Diebold, S.S., et al. (2004) Science 303:1529-1531; Hornung, V. et al. (2004) J. Immunol. 173:5935-5943); De Nardo, (2015) Cytokine 74: 181-189.

TLRs are a key means by which vertebrates recognize and mount an immune response to foreign molecules and also provide a means by which the innate and adaptive immune responses are linked (Akira, S. et al. (2001) Nature Immunol. 2:675-680; Medzhitov, R. (2001) Nature Rev. Immunol. 1:135-145). Some TLRs are located on the cell surface to detect and initiate a response to extracellular pathogens and other TLRs are located inside the cell to detect and initiate a response to intracellular pathogens.

TLR9 is known to recognize unmethylatcd CpG motifs in bacterial DNA and in synthetic oligonucleotides. (Hemmi, H. et al. (2000) Nature 408:740-745). Naturally occurring agonists of TLR9 have been shown to produce anti-tumor activity (e.g. tumor growth and angiogenesis) resulting in an effective anti-cancer response (e.g. anti-leukemia) (Smith, J.B. and Wickstrom, E. (1998) J. Natl. Cancer Inst. 90:1146-1154).

One of the most tantalizing prospects of cancer immunotherapy is the potential for longer-lasting cancer control. Activated immune cells retain a permanent memory of the cancer cells' unique protein marker, or antigen. If cancer reappears, the immune cells reactivate. However, many factors have been identified that cause the immune system to ignore cancer cells and thereby limit the effectiveness of cancer immunotherapies (Marabelle et al. (2013) J Clin Invest, 123(6):2447-2463; Mellman et al (2011) Nature 480(7378):480-489). Specifically, the immune system has numerous molecular brakes, or checkpoints, that function as endogenous inhibitory pathways in the immune system responsible for maintaining self-tolerance and modulating the degree of immune system response to minimize peripheral tissue damage. Additionally, tumor tissues have been shown to co-opt the checkpoint system to reduce the effectiveness of host immune response, resulting in inhibition of the immune system and tumor growth (see, e.g., Pardoll, 2012, Nature Reviews Cancer 12:252-64; Nirschl & Drake, 2013, Clin Cancer Res 19:4917-24).

Thus there is a need for therapies that keep the immune system engaged to improve efficacy of immunomodulatory therapies against tumor cells.

### BRIEF SUMMARY OF THE INVENTION

The invention provides methods of inducing an immune response to cancer comprising co-administering to a cancer patient one or more TLR9 agonists and one or more checkpoint inhibitors. Preferably, the one or more TLR9 agonists are administered to the patient via intratumoral (i.t.) administration. Preferably, the one or more TLR9 agonist is an immunomer.

Disclosed herein, in certain embodiments, is a method of treating a cancer in an individual in need thereof which comprises co-administering to a patient one or more TLR9 agonist and one or more checkpoint inhibitors. In some embodiments, the one or more TLR9 agonist are administered intratumorally. In some embodiments, the TLR9 agonists is an immunomer. In some embodiments, the immunomer is a compound selected from Table II. In some embodiments, the immune checkpoint inhibitor is an inhibitor of Programmed Death-Ligand 1 (PD-L1, also known as B7-H1, CD274), Programmed Death 1 (PD-1), CTLA-4, PD-L2 (B7-DC, CD273), LAG3, TIM3, 2B4, A2aR, B7H1, B7H3, B7H4, BTLA, CD2, CD27, CD28, CD30, CD40, CD70, CD80, CD86, CD137, CD160, CD226, CD276, DR3, GAL9, GITR, HAVCR2, HVEM, IDO1, IDO2, ICOS (inducible T cell costimulator), KIR, LAIR1, LIGHT, MARCO (macrophage receptor with collageneous structure), PS (phosphatidylserine), OX-40, SLAM, TIGHT, VISTA, VTCN1, or any combinations thereof. In some embodiments, the immune checkpoint inhibitor is an inhibitor of IDO1, CTLA4, PD-1, LAG3, PD-L1, TIM3, or combinations thereof. In some embodiments, the immune checkpoint inhibitor is an inhibitor of PD-L1. In some embodiments, the immune checkpoint inhibitor is an inhibitor of PD-1. In some embodiments, the immune checkpoint inhibitor is an inhibitor of CTLA-4. In some embodiments, the immune checkpoint inhibitor is an inhibitor of LAG3. In some embodiments, the immune checkpoint inhibitor is an inhibitor of TIM3. In some embodiments, the immune checkpoint inhibitor is an inhibitor of IDO1. In some embodiments, the one or more checkpoint inhibitors are administered by any suitable route. In some embodiments, the route of administration of the one or more checkpoint inhibitors is parenteral, mucosal delivery, oral, sublingual, transdermal, topical, inhalation, intranasal, aerosol, intratumoral, intraocular, intratracheal, intrarectal, intragastric, vaginal, by gene gun, dermal patch or in eye drop or mouthwash form. In some embodiments, the one or more TLR9 agonists and the one or more checkpoint inhibitors are each administered in a pharmaceutically effective amount. In some embodiments, the cancer is a solid tumor.

In some embodiments, the cancer is a hematologic cancer. In some embodiments, the hematologic cancer is a leukemia, a lymphoma, a myeloma, a non-Hodgkin's lymphoma, a Hodgkin's lymphoma, or a B-cell malignancy. In some embodiments, the hematologic cancer is a B-cell malignancy. In some embodiments, the B-cell malignancy is follicular lymphoma (FL), diffuse large B-cell lymphoma (DLBCL), mantle cell lymphoma (MCL), Waldenstrom's macroglobulinemia, multiple myeloma, extranodal marginal zone B cell lymphoma, nodal marginal zone B cell lymphoma, Burkitt's lymphoma, non-Burkitt high grade B cell lymphoma, primary mediastinal B-cell lymphoma (PMBL), immunoblastic large cell lymphoma, precursor B-lymphoblastic lymphoma, B cell prolymphocytic leukemia, lymphoplasmacytic lymphoma, splenic marginal zone lymphoma, plasma cell myeloma, plasmacytoma, mediastinal (thymic) large B cell lymphoma, intravascular large B cell lymphoma, primary effusion lymphoma, or lymphomatoid granulomatosis. In some embodiments, the B-cell malignancy is diffuse large B-cell lymphoma (DLBCL). In some embodiments, DLBCL is activated B-cell diffuse large B-cell lymphoma (ABC-DLBCL). In some embodiments, the B-cell malignancy is chronic lymphocytic leukemia (CLL), small lymphocytic lymphoma (SLL), B cell prolymphocytic leukemia (B-PLL), non-CLL/SLL lymphoma, mantle cell lymphoma, multiple myeloma, Waldenstrom's macroglobulinemia, or a combination thereof. In some embodiments, the B-cell malignancy is a relapsed or refractory B-cell malignancy. In some embodiments, the relapsed or refractory B-cell malignancy is diffuse large B-cell lymphoma (DLBCL). In some embodiments, the relapsed or refractory DLBCL is activated B-cell diffuse large B-cell lymphoma (ABC-DLBCL). In some embodiments, the relapsed or refractory B-cell malignancy is chronic lymphocytic leukemia (CLL), small lymphocytic lymphoma (SLL), B cell prolymphocytic leukemia (B-PLL), non-CLL/SLL lymphoma, mantle cell lymphoma, multiple myeloma, Waldenstrom's macroglobulinemia, or a combination thereof. In some embodiments, the B-cell malignancy is a metastasized B-cell malignancy. In some embodiments, the metastasized B-cell malignancy is diffuse large B-cell lymphoma (DLBCL), chronic lymphocytic leukemia (CLL), small lymphocytic lymphoma (SLL), B cell prolymphocytic leukemia (B-PLL), non-CLL/SLL lymphoma, mantle cell lymphoma, multiple myeloma, Waldenstrom's macroglobulinemia, or a combination thereof. In some embodiments, the cancer is a sarcoma, or carcinoma. In some embodiments, the cancer is selected from anal cancer; appendix cancer; bile duct cancer (i.e., cholangiocarcinoma); bladder cancer; breast cancer; cervical cancer; colon cancer; cancer of Unknown Primary (CUP); esophageal cancer; eye cancer; fallopian tube cancer; gastroenterological cancer; kidney cancer; liver cancer; lung cancer; medulloblastoma; melanoma; oral cancer; ovarian cancer; pancreatic cancer; parathyroid disease; penile cancer; pituitary tumor; prostate cancer; rectal cancer; skin cancer; stomach cancer; testicular cancer; throat cancer; thyroid cancer; uterine cancer; vaginal cancer; or vulvar cancer. In some embodiments, the cancer is selected from bladder cancer, breast cancer, colon cancer, gastroenterological cancer, kidney cancer, lung cancer, ovarian cancer, pancreatic cancer, prostate cancer, proximal or distal bile duct cancer, and melanoma. In some embodiments, the cancer is a breast cancer. In some embodiments, the breast cancer is ductal carcinoma *in situ,* lobular carcinoma *in situ,* invasive or infiltrating ductal carcinoma, invasive or infiltrating lobular carcinoma, inflammatory breast cancer, triple-negative breast cancer, paget disease of the nipple, phyllodes tumor, angiosarcoma or invasive breast carcinoma. In some embodiments, the cancer is a colon cancer. In some embodiments, the colon cancer is adenocarcinoma, gastrointestinal carcinoid tumors, gastrointestinal stromal tumors, primary colorectal lymphoma, leiomyosarcoma, melanoma, squamous cellcarcinoma, mucinous adenocarcinoma, or Signet ring cell adenocarcinoma. In some embodiments, the cancer is a relapsed or refractory cancer. In some embodiments, the relapsed or refractory cancer is selected from bladder cancer, breast cancer, colon cancer, gastroenterological cancer, kidney cancer, lung cancer, ovarian cancer, pancreatic cancer, prostate cancer, proximal or distal bile duct cancer, and melanoma. In some embodiments, the cancer is a metastasized cancer. In some embodiments, the metastasized cancer is selected from bladder cancer, breast cancer, colon cancer, gastroenterological cancer, kidney cancer, lung cancer, ovarian cancer, pancreatic cancer, prostate cancer, proximal or distal bile duct cancer, and melanoma.

In some embodiments, the immune checkpoint inhibitor is an antibody. In some embodiments, the immune checkpoint inhibitor is a monoclonal antibody.

In some embodiments, the use of a combination comprising of immune checkpoint inhibitor treatment and intratumoral administration of TLR9 agonist for the treatment of a cancer further comprises administering an additional anticancer agent. In some embodiments, the additional anticancer agent is selected from among a chemotherapeutic agent or radiation therapy. In some embodiments, the chemotherapeutic agent is selected from among chlorambucil, ifosfamide, doxorubicin, mesalazine, thalidomide, lenalidomide, temsirolimus, everolimus, fludarabine, fostamatinib, paclitaxel, docetaxel, ofatumumab, rituximab, dexamethasone, prednisone, CAL-101, ibritumomab, tositumomab, bortezomib, pentostatin, endostatin, or a combination thereof.

### BRIEF DESCRIPTION OF THE DRAWINGS

The foregoing and other objects, features and advantages of the invention will be apparent from the following more particular description of preferred embodiments of the invention, as illustrated in the accompanying drawings in which like reference characters refer to the same parts throughout the different views. The drawings are not necessarily to scale, emphasis instead being placed upon illustrating the principles of the invention.
FIG. 1 is a synthetic scheme for the linear synthesis of immunomers. DMTr = 4,4'-dimethoxytrityl; CE = cyanoethyl.
FIG. 2 is an example of a synthetic scheme for the parallel synthesis of immunomers. DMTr = 4,4'-dimethoxytrityl; CE = cyanoethyl.
FIG. 3A and FIG. 3B demonstrates that intratumoral administration of TLR9 agonist induced potent antitumor activity and increase CD3+ TIL infiltration compared to subcutaneous administration.
FIG. 4A and FIG. 4B demonstrates that intratumoral administration of TLR9 agonist induced potent antitumor activity on both local and distant tumors in A20 lymphoma model.
FIG. 5A and FIG. 5B demonstrates that intratumoral administration of TLR9 agonist induced potent antitumor activity on both local and distant tumors in CT26 colon carcinoma model.
FIG. 6A and FIG. 6B demonstrates that intratumoral administration of TLR9 agonist induced potent antitumor activity on both local and distant tumors in B16 melanoma model.
FIG. 7A through FIG. 7D demonstrates that combination of anti-CTLA4 mAb treatment and intratumoral injections of TLR9 agonist lead to tumor growth inhibition on directly treated tumor nodules.
FIG. 8A and FIG. 8B demonstrates that anti-CTLA4 mAb treatment and intratumoral administered TLR9 agonist leads to regression of systemic lung metastasis.
FIG. 9A through FIG. 9D demonstrates that combined intratumorally administered TLR9 agonist and anti-CTLA4 mAb therapy enhances T cell infiltration in lung metastatic tumors. FIG. 9A shows that a few T cells are present in the tumor tissues bordering normal tissue in the PBS treated group. FIG. 9B and FIG 9C show increased T cells infiltration into tumor tissues; however, most abundant T cell infiltration is present in tumors from mice receiving combined treatment of TLR9 agonist and CTLA-4 mAb. (CD3 IHC stain x400)
FIG. 10A and FIG. 10B demonstrate that anti-CTLA4 mAb treatment and intratumoral injections of TLR9 agonist on a treated local tumor lead to potent antitumor effects to both local and distant tumors.
FIG. 11A through FIG. 11E demonstrate that anti-CTLA4 mAb and intratumoral injections of TLR9 agonist increases T lymphocyte infiltration into tumor tissues. While few CD3+ cells present in the tumor tissue bordering normal tissue from PBS (vehicle) injected mice, a large number of CD+3 cells are presented in the tumor tissue from mice treated with TLR9 agonists or CTLA-mAb. However, most abundant CD3+ cells are present in tumors from mice receiving combined treatment of TLR9 agonist and CTLA-4 mAb.
FIG. 12A through FIG. 12D demonstrates that combination of anti-PD-1 mAb treatment and intratumoral injections of TLR9 agonist lead to tumor growth inhibition on directly treated tumor nodules.
FIG. 13A and FIG. 13B demonstrate that anti-PD-1 treatment and intratumoral administered TLR9 agonist leads to regression of systemic lung metastasis.
FIG. 14A through FIG. 14E demonstrates that combination of anti-IDOl inhibitor treatment and intratumoral injections of TLR9 agonist lead to tumor growth inhibition on directly treated tumor nodules.
FIG. 15A and FIG. 15B demonstrate that anti-IDO1 treatment and intratumoral administered TLR9 agonist leads to regression of systemic lung metastasis.
FIG. 16A through FIG. 16D demonstrate that anti-IDOl treatment and intratumoral administered TLR9 agonist leads to systemic metastatic tumor suppression. FIG. 16A shows that tumor nodules are infiltrating into most of the lung tissues in the PBS treated group. FIG. 16B and FIG 16C show tumor nodules are smaller than that of the PBS group, and present on the edge of the lung tissues for TLR9 agonist group; however, most of lung tissues are clear of tumor nodules from mice receiving combined treatment of TLR9 agonist and IDO.
FIG. 17A through FIG. 17D demonstrates that treatment with TLR9 agonist and IDO-1 inhibitor increases CD3+ T cell infiltrations in lung metastatic tumors.

### DETAILED DESCRIPTION OF THE PREFERRED EMBODIMENTS

The present invention generally relates to the field of oncology, and more specifically the use of immunotherapy in the treatment or prevention of cancer. Preferably, the invention provides the co-administration of one or more TLR9 agonists and one or more checkpoint inhibitors. These agents may be used to induce or enhance the immune response against disease-associated antigens, such as tumor-associated antigens (TAAs) and enhance overall efficacy of treatment.

Without being held to any particular theory, Toll-like receptors (TLRs) are believed to play a central role in the innate immune system, the body's first line of defense against invading pathogens, as well as damaged or dysfunctional cells including cancer cells. Intratumoral administration of TLR9 agonists is shown to have potent anti-tumor activity; however, despite the promise of a TLR9 agonist monotherapy, the resulting immune response induced immune system suppression pathways including immune checkpoints that diminish the efficacy of the TLR9 agonists. Therefore a combination therapy seems necessary.

The innate immune system is also involved in activating the adaptive immune system, which marshals highly specific immune responses to target pathogens or tissue. However, cancer cells may exploit regulatory checkpoint pathways to avoid being recognized by the immune system, thereby shielding the tumor from immune attack.

Currently, checkpoint inhibitors are being designed to block these immune checkpoints thereby enabling the immune system to recognize tumor cells and allowing a sustained immunotherapy response. While monotherapy treatments with checkpoint inhibitors have shown some promising results, these results were only shown in patients that were PD-L1 positive. Additionally, a potential drawback to the use of checkpoint inhibitors as a monotherapy is the generation of autoimmune toxicities.

Intratumoral administration of TLR9 agonists results in changes in the tumor microenvironment in both treated and distant tumors, as demonstrated by modulation of immune checkpoint gene expression. In this setting, intratumoral TLR9 agonist administration may increase the tumor-infiltrating lymphocytes (TILs); and potentiate anticancer activity of checkpoint inhibitors in the injected tumor as well as systemically. Therefore, intratumoral administration of TLR9 agonists can sensitize the tumor microenvironment for combination with one or more checkpoint inhibitors.

All publications cited herein reflect the level of skill in the art and are hereby incorporated by reference in their entirety. Any conflict between the teachings of these references and this specification shall be resolved in favor of the latter.

### DEFINITIONS

The term "2'-substituted nucleoside" or "2'-substituted arabinoside" generally includes nucleosides or arabinonucleosides in which the hydroxyl group at the 2' position of a pentose or arabinose moiety is substituted to produce a 2'-substituted or 2'-O-substituted ribonucleoside. In certain embodiments, such substitution is with a lower hydrocarbyl group containing 1-6 saturated or unsaturated carbon atoms, with a halogen atom, or with an aryl group having 6-10 carbon atoms, wherein such hydrocarbyl, or aryl group may be unsubstituted or may be substituted, e.g., with halo, hydroxy, trifluoromethyl, cyano, nitro, acyl, acyloxy, alkoxy, carboxyl, carboalkoxy, or amino groups. Examples of 2'-O-substituted ribonucleosides or 2'-O-substituted-arabinosides include, without limitation 2'-amino, 2'-fluoro, 2'-allyl, 2'-O-alkyl and 2'-propargyl ribonucleosides or arabinosides, 2'-O-methylribonucleosides or 2'-O-methylarabinosides and 2'-O-methoxyethoxyribonucleosides or 2'-O-methoxyethoxyarabinosides.

The term "3' ", when used directionally, generally refers to a region or position in a polynucleotide or oligonucleotide 3' (toward the 3' position of the oligonucleotide) from another region or position in the same polynucleotide or oligonucleotide.

The term " 5' ", when used directionally, generally refers to a region or position in a polynucleotide or oligonucleotide 5' (toward the 5' position of the oligonucleotide) from another region or position in the same polynucleotide or oligonucleotide.

The term "about" generally means that the exact number is not critical. Thus, the number of nucleoside residues in the oligonucleotides is not critical, and oligonucleotides having one or two fewer nucleoside residues, or from one to several additional nucleoside residues are contemplated as equivalents of each of the embodiments described above.

The term "adjuvant" generally refers to a substance which, when added to an immunogenic agent such as vaccine or antigen, enhances or potentiates an immune response to the agent in the recipient host upon exposure to the mixture.

The antibodies for use in the present invention include, but are not limited to, monoclonal antibodies, synthetic antibodies, polyclonal antibodies, multispecific antibodies, human antibodies, humanized antibodies, chimeric antibodies, single-chain Fvs (scFv) (including bi-specific scFvs), single chain antibodies, Fab fragments, F(ab') fragments, disulfide-linked Fvs (sdFv), and epitope-binding fragments of any of the above. In particular, antibodies for use in the present invention include immunoglobulin molecules and immunologically active portions of immunoglobulin molecules, i.e., molecules that contain a binding site for an immune checkpoint molecule that immunospecifically bind to the immune checkpoint molecule. The immunoglobulin molecules for use in the invention can be of any type {e.g., IgG, IgE, IgM, IgD, IgA and IgY), class {e.g., IgG1, IgG2, IgG3, IgG4, IgA1 and IgA2) or subclass of immunoglobulin molecule. Preferably, the antibodies for use in the invention are IgG, more preferably, IgG1. An antibody against an immune checkpoint molecule suitable for use with the methods disclosed herein may be from any animal origin including birds and mammals {e.g., human, murine, donkey, sheep, rabbit, goat, guinea pig, camel, horse, shark or chicken). Preferably, the antibodies are human or humanized monoclonal antibodies. As used herein, "human" antibodies include antibodies having the amino acid sequence of a human immunoglobulin and include antibodies isolated from human immunoglobulin libraries or from mice or other animals that express antibodies from human genes. An antibody against an immune checkpoint molecule suitable for use with the methods disclosed herein may be monospecific, bispecific, trispecific or of greater multispecificity. Multispecific antibodies may immunospecifically bind to different epitopes of a polypeptide or may immunospecifically bind to both a polypeptide as well as a heterologous epitope, such as a heterologous polypeptide or solid support material.

The term "agonist" generally refers to a substance that binds to a receptor of a cell and induces a response. Such response may be an increase in the activity mediated by the receptor. An agonist often mimics the action of a naturally occurring substance such as a ligand.

The term "antagonist" or "inhibitor" generally refers to a substance that can bind to a receptor, but does not produce a biological response upon binding. The antagonist or inhibitor can block, inhibit, or attenuate the response mediated by an agonist and may compete with agonist for binding to a receptor. Such antagonist or inhibitory activity may be reversible or irreversible.

The term "antigen" generally refers to a substance that is recognized and selectively bound by an antibody or by a T cell antigen receptor. Antigens may include but are not limited to peptides, proteins, nucleosides, nucleotides and combinations thereof. Antigens may be natural or synthetic and generally induce an immune response that is specific for that antigen.

The term "cancer" generally refers to, without limitation, any malignant growth or tumor caused by abnormal or uncontrolled cell proliferation and/or division. Cancers may occur in humans and/or animals and may arise in any and all tissues. Treating a patient having cancer with the invention may include administration of a compound, pharmaceutical formulation or vaccine according to the invention such that the abnormal or uncontrolled cell proliferation and/or division is affected.

The term "carrier" generally encompasses any excipient, diluent, filler, salt, buffer, stabilizer, solubilizer, oil, lipid, lipid containing vesicle, microspheres, liposomal encapsulation, or other material well known in the art for use in pharmaceutical formulations. It will be understood that the characteristics of the carrier, excipient, or diluent will depend on the route of administration for a particular application. The preparation of pharmaceutically acceptable formulations containing these materials is described in, *e.g.,* Remington's Pharmaceutical Sciences, 18th Edition, ed. A. Gennaro, Mack Publishing Co., Easton, PA, 1990.

The term "pharmaceutically acceptable" or "physiologically acceptable" generally refers to a material that does not interfere with the effectiveness of a compound according to the invention, and that is compatible with a biological system such as a cell, cell culture, tissue, or organism. Preferably, the biological system is a living organism, such as a vertebrate.

The term "co-administration", "co-administering", or "co-administered" generally refers to the administration of at least two different therapeutic agents sufficiently close in time. Such administration may be done in any order, including simultaneous administration, as well as temporally spaced order from a few seconds up to several days apart. Such administration may also include more than a single administration of one agent and/or independently the other agent. The administration of the agents may be by the same or different routes.

The terms "enhance" or "enhancing" means to increase or prolong either in potency or duration a desired effect. By way of example, "enhancing" the effect of therapeutic agents refers to the ability to increase or prolong, either in potency or duration, the effect of therapeutic agents on during treatment of a disease, disorder or condition. An "enhancing-effective amount," as used herein, refers to an amount adequate to enhance the effect of a therapeutic agent in the treatment of a disease, disorder or condition. When used in a patient, amounts effective for this use will depend on the severity and course of the disease, disorder or condition, previous therapy, the patient's health status and response to the drugs, and the judgment of the treating physician.

The term an "effective amount" generally refers to an amount sufficient to affect a desired biological effect, such as a beneficial result. Thus, an "effective amount" will depend upon the context in which it is being administered. A effective amount may be administered in one or more prophylactic or therapeutic administrations.

The term "in combination with" generally means administering a first agent and another agent useful for treating the disease or condition.

The term "individual", "patient", or "subject" are used interchangeably and generally refers to a mammal, such as a human. Mammals generally include, but are not limited to, humans, non-human primates, rats, mice, cats, dogs, horses, cattle, cows, pigs, sheep and rabbits.

The term "kinase inhibitor" generally refers to molecules that antagonize or inhibit phosphorylation-dependent cell signaling and/or growth pathways in a cell. Kinase inhibitors may be naturally occurring or synthetic and include small molecules that have the potential to be administered as oral therapeutics. Kinase inhibitors have the ability to rapidly and specifically inhibit the activation of the target kinase molecules. Protein kinases are attractive drug targets, in part because they regulate a wide variety of signaling and growth pathways and include many different proteins. As such, they have great potential in the treatment of diseases involving kinase signaling, including cancer, cardiovascular disease, inflammatory disorders, diabetes, macular degeneration and neurological disorders. Examples of kinase inhibitors include sorafenib (NEXAVAR^{®}), SUTENT^{®}, dasatinib, ZACT1MA^{™}, TYKERB^{™}, ibrutinib (IMBRUVICA^{®}), and STI571.

The term "linear synthesis" generally refers to a synthesis that starts at one end of an oligonucleotide and progresses linearly to the other end. Linear synthesis permits incorporation of either identical or non-identical (in terms of length, base composition and/or chemical modifications incorporated) monomeric units into an oligonucleotide.

The term "modified nucleoside" generally is a nucleoside that includes a modified heterocyclic base, a modified sugar moiety, or any combination thereof. In some embodiments, the modified nucleoside is a non-natural pyrimidine or purine nucleoside, as herein described. For purposes of the invention, a modified nucleoside, a pyrimidine or purine analog or non-naturally occurring pyrimidine or purine can be used interchangeably and refers to a nucleoside that includes a non-naturally occurring base and/or non-naturally occurring sugar moiety. For purposes of the invention, a base is considered to be non-natural if it is not guanine, cytosine, adenine, thymine or uracil.

The term "linker" generally refers to any moiety that can be attached to an oligonucleotide by way of covalent or non-covalent bonding through a sugar, a base, or the backbone. The linker can be used to attach two or more nucleosides or can be attached to the 5' and/or 3' terminal nucleotide in the oligonucleotide. In certain embodiments of the invention, such linker may be a non-nucleotidic linker.

The term "non-nucleotidic linker" generally refers to a chemical moiety other than a nucleotidic linkage that can be attached to an oligonucleotide by way of covalent or non-covalent bonding. Preferably such non-nucleotidic linker is from about 2 angstroms to about 200 angstroms in length, and may be either in a cis or trans orientation.

The term "nucleotidic linkage" generally refers to a chemical linkage to join two nucleosides through their sugars (e.g. 3'-3', 2'-3', 2'-5', 3'-5') consisting of a phosphorous atom and a charged, or neutral group (e.g., phosphodiester, phosphorothioate or phosphorodithioate) between adjacent nucleosides.

The term "oligonucleotide" refers to a polynucleoside formed from a plurality of linked nucleoside units. The nucleoside units may be part of or may be made part of viruses, bacteria, cell debris, siRNA or microRNA. Such oligonucleotides can also be obtained from existing nucleic acid sources, including genomic or cDNA, but are preferably produced by synthetic methods. In preferred embodiments each nucleoside unit includes a heterocyclic base and a pentofuranosyl, trehalose, arabinose, 2'-deoxy-2'-substituted nucleoside, 2'-deoxy-2'-substituted arabinose, 2'-O-substitutedarabinose or hexose sugar group. The nucleoside residues can be coupled to each other by any of the numerous known internucleoside linkages. Such internucleoside linkages include, without limitation, phosphodiester, phosphorothioate, phosphorodithioate, alkylphosphonate, alkylphosphonothioate, phosphotriester, phosphoramidate, siloxane, carbonate, carboalkoxy, acetamidate, carbamate, morpholino, borano, thioether, bridged phosphoramidate, bridged methylene phosphonate, bridged phosphorothioate, and sulfone internucleoside linkages. The term "oligonucleotide-based compound" also encompasses polynucleosides having one or more stereospecific internucleoside linkage (e.g., (*Rp*)*-* or (*Sₚ*)-phosphorothioate, alkylphosphonate, or phosphotriester linkages). As used herein, the terms "oligonucleotide" and "dinucleotide" are expressly intended to include polynucleosides and dinucleosides having any such internucleoside linkage, whether or not the linkage comprises a phosphate group. In certain preferred embodiments, these internucleoside linkages may be phosphodiester, phosphorothioate or phosphorodithioate linkages, or combinations thereof.

The term "peptide" generally refers to polypeptides that are of sufficient length and composition to affect a biological response, e.g., antibody production or cytokine activity whether or not the peptide is a hapten. The term "peptide" may include modified amino acids (whether or not naturally or non-naturally occurring), where such modifications include, but are not limited to, phosphorylation, glycosylation, pegylation, lipidization and methylation.

The term "treatment" generally refers to an approach intended to obtain a beneficial or desired result, which may include alleviation of symptoms, or delaying or ameliorating a disease progression.

Disclosed herein, in certain embodiments, is a method of treating a cancer in an individual in need thereof which comprises co-administering to a patient one or more TLR9 agonist and one or more checkpoint inhibitors. In some embodiments, the one or more TLR9 agonist are administered intratumorally. In some embodiments, the TLR9 agonists is an immunomer. In some embodiments, the immunomer is a compound selected from Table II. In some embodiments, the immune checkpoint inhibitor is an inhibitor of Programmed Death-Ligand 1 (PD-L1, also known as B7-H1, CD274), Programmed Death 1 (PD-1), CTLA-4, PD-L2 (B7-DC, CD273), LAG3, TIM3, 2B4, A2aR, B7H1, B7H3, B7H4, BTLA, CD2, CD27, CD28, CD30, CD40, CD70, CD80, CD86, CD137, CD160, CD226, CD276, DR3, GAL9, G1TR, HAVCR2, HVEM, IDO1, IDO2, ICOS (inducible T cell costimulator), KIR, LAIR1, LIGHT, MARCO (macrophage receptor with collageneous structure), PS (phosphatidylserine), OX-40, SLAM, TIGHT, VISTA, VTCN1, or any combinations thereof. In some embodiments, the immune checkpoint inhibitor is an inhibitor of IDO1, CTLA4, PD-1, LAG3, PD-L1, TIM3, or combinations thereof. In some embodiments, the immune checkpoint inhibitor is an inhibitor of PD-L1. In some embodiments, the immune checkpoint inhibitor is an inhibitor of PD-1. In some embodiments, the immune checkpoint inhibitor is an inhibitor of CTLA-4. In some embodiments, the immune checkpoint inhibitor is an inhibitor of LAG3. In some embodiments, the immune checkpoint inhibitor is an inhibitor of TIM3. In some embodiments, the immune checkpoint inhibitor is an inhibitor of IDO1. In some embodiments, the one or more checkpoint inhibitors are administered by any suitable route. In some embodiments, the route of administration of the one or more checkpoint inhibitors is parenteral, mucosal delivery, oral, sublingual, transdermal, topical, inhalation, intranasal, aerosol, intratumoral, intraocular, intratracheal, intrarectal, intragastric, vaginal, by gene gun, dermal patch or in eye drop or mouthwash form. In some embodiments, the one or more TLR9 agonists and the one or more checkpoint inhibitors are each administered in a pharmaceutically effective amount. In some embodiments, the cancer is a solid tumor. In some embodiments, the cancer is a hematologic cancer.

Disclosed herein, in certain embodiments, is a method of treating a solid tumor in an individual in need thereof which comprises co-administering to a patient one or more TLR9 agonist and one or more checkpoint inhibitors. In some embodiments, the one or more TLR9 agonist are administered intratumorally. In some embodiments, the TLR9 agonists is an immunomer. In some embodiments, the immunomer is a compound selected from Table II. In some embodiments, the immune checkpoint inhibitor is an inhibitor of Programmed Death-Ligand 1 (PD-L1, also known as B7-H1, CD274), Programmed Death 1 (PD-1), CTLA-4, PD-L2 (B7-DC, CD273), LAG3, TIM3, 2B4, A2aR, B7H1, B7H3, B7H4, BTLA, CD2, CD27, CD28, CD30, CD40, CD70, CD80, CD86, CD137, CD160, CD226, CD276, DR3, GAL9, GITR, HAVCR2, HVEM, IDO1, IDO2, ICOS (inducible T cell costimulator), KIR, LAIR1, LIGHT, MARCO (macrophage receptor with collageneous structure), PS (phosphatidylserine), OX-40, SLAM, TIGHT, VISTA, VTCN1, or any combinations thereof. In some embodiments, the immune checkpoint inhibitor is an inhibitor of IDO1, CTLA4, PD-1, LAG3, PD-L1, TIM3, or combinations thereof. In some embodiments, the immune checkpoint inhibitor is an inhibitor of PD-L1. In some embodiments, the immune checkpoint inhibitor is an inhibitor of PD-1. In some embodiments, the immune checkpoint inhibitor is an inhibitor of CTLA-4. In some embodiments, the immune checkpoint inhibitor is an inhibitor of LAG3. In some embodiments, the immune checkpoint inhibitor is an inhibitor of TIM3. In some embodiments, the immune checkpoint inhibitor is an inhibitor of IDO1. In some embodiments, the one or more checkpoint inhibitors are administered by any suitable route. In some embodiments, the route of administration of the one or more checkpoint inhibitors is parenteral, mucosal delivery, oral, sublingual, transdermal, topical, inhalation, intranasal, aerosol, intratumoral, intraocular, intratracheal, intrarectal, intragastric, vaginal, by gene gun, dermal patch or in eye drop or mouthwash form. In some embodiments, the one or more TLR9 agonists and the one or more checkpoint inhibitors are each administered in a pharmaceutically effective amount. In some embodiments, the solid tumor is a sarcoma or carcinoma. In some embodiments, the solid tumor is a sarcoma. In some embodiments, the solid tumor is a carcinoma.

In some embodiments, the solid tumor is a relapsed or refractory solid tumor. In some embodiments, the relapsed or refractory solid tumor is a sarcoma or carcinoma. In some embodiments, the relapsed or refractory solid tumor is a sarcoma. In some embodiments, the relapsed or refractory solid tumor is a carcinoma.

In some embodiments, the solid tumor is a metastasized solid tumor. In some embodiments, the metastasized solid tumor is a sarcoma or carcinoma. In some embodiments, the metastasized solid tumor is a sarcoma. In some embodiments, the metastasized solid tumor is a carcinoma.

In some embodiments, the sarcoma is selected from alveolar rhabdomyosarcoma; alveolar soft part sarcoma; ameloblastoma; angiosarcoma; chondrosarcoma; chordoma; clear cell sarcoma of soft tissue; dedifferentiated liposarcoma; desmoid; desmoplastic small round cell tumor; embryonal rhabdomyosarcoma; epithelioid fibrosarcoma; epithelioid hemangioendothelioma; epithelioid sarcoma; esthesioneuroblastoma; Ewing sarcoma; extrarenal rhabdoid tumor; extraskeletal myxoid chondrosarcoma; extraskeletal osteosarcoma; fibrosarcoma; giant cell tumor; hemangiopericytoma; infantile fibrosarcoma; inflammatory myofibroblastic tumor; Kaposi sarcoma; leiomyosarcoma of bone; liposarcoma; liposarcoma of bone; malignant fibrous histiocytoma (MFH); malignant fibrous histiocytoma (MFH) of bone; malignant mesenchymoma; malignant peripheral nerve sheath tumor; mesenchymal chondrosarcoma; myxofibrosarcoma; myxoid liposarcoma; myxoinflammatory fibroblastic sarcoma; neoplasms with perivascular epitheioid cell differentiation; osteosarcoma; parosteal osteosarcoma; neoplasm with perivascular epitheioid cell differentiation; periosteal osteosarcoma; pleomorphic liposarcoma; pleomorphic rhabdomyosarcoma; PNET/extraskeletal Ewing tumor; rhabdomyosarcoma; round cell liposarcoma; small cell osteosarcoma; solitary fibrous tumor; synovial sarcoma; telangiectatic osteosarcoma.

In some embodiments, the carcinoma is selected from an adenocarcinoma, squamous cell carcinoma, adenosquamous carcinoma, anaplastic carcinoma, large cell carcinoma, or small cell carcinoma. In some embodiments, the carcinoma is selected from anal cancer; appendix cancer; bile duct cancer (i.e., cholangiocarcinoma); bladder cancer; breast cancer; cervical cancer; colon cancer; cancer of Unknown Primary (CUP); esophageal cancer; eye cancer; fallopian tube cancer; gastroenterological cancer; kidney cancer; liver cancer; lung cancer; medulloblastoma; melanoma; oral cancer; ovarian cancer; pancreatic cancer; parathyroid disease; penile cancer; pituitary tumor; prostate cancer; rectal cancer; skin cancer; stomach cancer; testicular cancer; throat cancer; thyroid cancer; uterine cancer; vaginal cancer; or vulvar cancer. In some embodiments, the carcinoma is breast cancer. In some embodiments, the breast cancer is invasive ductal carcinoma, ductal carcinoma *in situ,* invasive lobular carcinoma, or lobular carcinoma *in situ.* In some embodiments, the carcinoma is pancreatic cancer. In some embodiments, the pancreatic cancer is adenocarcinoma, or islet cell carcinoma. In some embodiments, the carcinoma is colorectal (colon) cancer. In some embodiments, the colorectal cancer is adenocarcinoma. In some embodiments, the solid tumor is a colon polyp. In some embodiments, the colon polyp is associated with familial adenomatous polyposis. In some embodiments, the carcinoma is bladder cancer. In some embodiments, the bladder cancer is transitional cell bladder cancer, squamous cell bladder cancer, or adenocarcinoma. In some embodiments, the bladder cancer is encompassed by the genitourinary tract cancers. In some embodiments, the genitourinary tract cancers also encompass kidney cancer, prostate cancer, and cancers associated with the reproductive organs. In some embodiments, the carcinoma is lung cancer. In some embodiments, the lung cancer is a non-small cell lung cancer. In some embodiments, the non-small cell lung cancer is adenocarcinoma, squamous-cell lung carcinoma, or large-cell lung carcinoma. In some embodiments, the lung cancer is a small cell lung cancer. In some embodiments, the carcinoma is prostate cancer. In some embodiments, the prostate cancer is adenocarcinoma or small cell carcinoma. In some embodiments, the carcinoma is ovarian cancer. In some embodiments, the ovarian cancer is epithelial ovarian cancer. In some embodiments, the carcinoma is bile duct cancer. In some embodiments, the bile duct cancer is proximal bile duct carcinoma or distal bile duct carcinoma.

In some embodiments, the solid tumor is selected from alveolar soft part sarcoma, bladder cancer, breast cancer, colorectal (colon) cancer, Ewing's bone sarcoma, gastroenterological cancer, head and neck cancer, kidney cancer, leiomyosarcoma, lung cancer, melanoma, osteosarcoma, ovarian cancer, pancreatic cancer, prostate cancer, proximal or distal bile duct cancer, and neuroblastoma. In some embodiments, the solid tumor is prostate cancer. In some embodiments, the solid tumor is breast cancer. In some embodiments, the solid tumor is lung cancer. In some embodiments, the solid tumor is colorectal (colon) cancer. In some embodiments, the solid tumor is gastroenterological cancer. In some embodiments, the solid tumor is melanoma. In some embodiments, the solid tumor is lung cancer. In some embodiments, the solid tumor is kidney cancer. In some embodiments, the solid tumor is head and neck cancer. In some embodiments, the solid tumor is proximal or distal bile duct cancer. In some embodiments, the solid tumor is alveolar soft part sarcoma. In some embodiments, the solid tumor is Ewing's bone sarcoma. In some embodiments, the solid tumor is bladder cancer. In some embodiments, the solid tumor is ovarian cancer. In some embodiments, the solid tumor is leiomyosarcoma. In some embodiments, the solid tumor is osteosarcoma. In some embodiments, the solid tumor is neuroblastoma.

In some embodiments, the breast cancer is ductal carcinoma *in situ* (intraductal carcinoma), lobular carcinoma *in situ,* invasive (or infiltrating) ductal carcinoma, invasive (or infiltrating) lobular carcinoma, inflammatory breast cancer, triple-negative breast cancer, paget disease of the nipple, phyllodes tumor, angiosarcoma or invasive breast carcinoma. In some embodiments, the invasive breast carcinoma is further categorized into subtypes. In some embodiments, the subtypes include adenoid cystic (or adenocystic) carcinoma, low-grade adenosquamous carcinoma, medullary carcinoma, mucinous (or colloid) carcinoma, papillary carcinoma, tubular carcinoma, metaplastic carcinoma, micropapillary carcinoma or mixed carcinoma.

In some embodiments, the breast cancer is classified according to stages or how far the tumor cells have spread within the breast tissues and to other portions of the body. In some embodiments, there are five stages of breast cancer, Stage 0-IV. In some embodiments, Stage 0 breast cancer refers to non-invasive breast cancers or that there are no evidence of cancer cells or abnormal non-cancerous cells breaking out of the origin site. In some embodiments, Stage I breast cancer refers to invasive breast cancer in which the cancer cells have invaded into surrounding tissues. In some embodiments, Stage I is subclassified into Stage IA and IB, in which Stage IA describes tumor measures up to 2 cm with no spread of cancer cells. Stage IB describes absence of tumor in breast but have small lumps of cancer cells between 0.2 mm to 2 mm within the lymph nodes. In some embodiments, Stage II breast cancer is further subdivided into Stage IIA and IIB. In some embodiments, Stage IIA describes tumor between 2 cm to 5 cm in breast only, or absence of tumor in breast but with cancer between 2 mm to 2 cm in axillary lymph nodes. In some embodiments, Stage IIB describes tumor larger than 5 cm in breast only, or tumor between 2 cm to 5 cm in breast with presence of small tumors from 0.2 mm to 2 mm in axillary lymph nodes. In some embodiments, Stage III breast cancer is further subdivided into Stage IIIA, IIIB, and IIIC. In some embodiments, Stage IIIA describes absence of tumor or tumor greater than 5 cm in breast with small tumors in 4-9 axillary lymph nodes or small tumors 0.2 mm-2 mm in size in axillary lymph nodes. In some embodiments, Stage IIIB describes tumor spreading into the chest wall or skin of the breast causing swelling or ulcer and with presence of tumor in up to 9 axillary lymph nodes. In some embodiments, inflammatory breast cancer is also considered as Stage IIIB. In some embodiments, Stage IIIC describes absence of tumor or tumor spreading into the chest wall or to the skin of the breast, with tumor present in 10 or more axillary lymph nodes. In some embodiments, Stage IV breast cancer refers to invasive breast cancer that has metastasized into the lymph nodes and other portions of the body.

In some embodiments, the colon cancer is a colorectal cancer. As used herein and throughout, colon cancer is used interchangeably with colorectal cancer. In some embodiments, colorectal (colon) cancer refers to rectal cancer. In some embodiments, the colon cancer is adenocarcinoma, gastrointestinal carcinoid tumors, gastrointestinal stromal tumors, primary colorectal lymphoma, leiomyosarcoma, melanoma, or squamous cell-carcinoma. In some embodiments, adenocarcinoma is a mucinous adenocarcinoma or a Signet ring cell adenocarcinoma.

In some embodiments, the colon cancer is classified according to stages or how far they have spread through the walls of the colon and rectum. In some embodiments, there are five stages of colon cancer, Stage 0-IV. In some embodiments, Stage 0 colon cancer refers to the very early stage of cancer. In some embodiments, Stage I colon cancer refers to when the cancer has spread beyond the innermost lining of the colon to the second and third layers and also involves the inside wall of the colon. In some embodiments, Stage II colon cancer refers to when the tumor has extended through the muscular wall but has not yet spread into the lymph nodes. In some embodiments, Stage III colon cancer refers to when the tumor has metastasized the colon into one or more lymph nodes. In some embodiments, Stage IV colon cancer refers to when the tumor has metastasized to other parts of the body. In some embodiments, there are two stages of rectal cancer, classified as Stage 0 and Stage I. In some embodiments, Stage 0 rectal cancer refers to when the tumor is located only on the inner lining of the rectum. In some embodiments, Stage I refers to when the tumor has advanced through the inner lining of the rectum but not yet reach past the muscular wall.

In some embodiments, the use of a combination comprising of immune checkpoint inhibitor treatment and intratumoral administration of TLR9 agonist for the treatment of a cancer further comprises administering an additional anticancer agent. In some embodiments, the additional anticancer agent is selected from among a chemotherapeutic agent or radiation therapy. In some embodiments, the chemotherapeutic agent is selected from among chlorambucil, ifosfamide, doxorubicin, mesalazine, thalidomide, lenalidomide, temsirolimus, everolimus, fludarabine, fostamatinib, paclitaxel, docetaxel, ofatumumab, rituximab, dexamethasone, prednisone, CAL-101, ibritumomab, tositumomab, bortezomib, pentostatin, endostatin, or a combination thereof.

Disclosed herein, in certain embodiments, is a method of treating a hematologic cancer in an individual in need thereof which comprises co-administering to a patient one or more TLR9 agonists and one or more checkpoint inhibitors. In some embodiments, the one or more TLR9 agonist are administered intratumorally. In some embodiments, the TLR9 agonists is an immunomer. In some embodiments, the immunomer is a compound selected from Table II. In some embodiments, the immune checkpoint inhibitor is an inhibitor of Programmed Death-Ligand 1 (PD-L1, also known as B7-H1, CD274), Programmed Death 1 (PD-1), CTLA-4, PD-L2 (B7-DC, CD273), LAG3, TIM3, 2B4, A2aR, B7H1, B7H3, B7H4, BTLA, CD2, CD27, CD28, CD30, CD40, CD70, CD80, CD86, CD137, CD160, CD226, CD276, DR3, GAL9, GITR, HAVCR2, HVEM, IDO1, ID02, ICOS (inducible T cell costimulator), KIR, LAIR1, LIGHT, MARCO (macrophage receptor with collageneous structure), PS (phosphatidylserine), OX-40, SLAM, TIGHT, VISTA, VTCN1, or any combinations thereof. In some embodiments, the immune checkpoint inhibitor is an inhibitor of IDO1, CTLA4, PD-1, LAG3, PD-L1, TIM3, or combinations thereof. In some embodiments, the immune checkpoint inhibitor is an inhibitor of PD-L1. In some embodiments, the immune checkpoint inhibitor is an inhibitor of PD-1. In some embodiments, the immune checkpoint inhibitor is an inhibitor of CTLA-4. In some embodiments, the immune checkpoint inhibitor is an inhibitor of LAG3. In some embodiments, the immune checkpoint inhibitor is an inhibitor of TIM3. In some embodiments, the one or more checkpoint inhibitors are administered by any suitable route. In some embodiments, the route of administration of the one or more checkpoint inhibitors is parenteral, mucosal delivery, oral, sublingual, transdermal, topical, inhalation, intranasal, aerosol, intratumoral, intraocular, intratracheal, intrarectal, intragastric, vaginal, by gene gun, dermal patch or in eye drop or mouthwash form. In some embodiments, the one or more TLR9 agonists and the one or more checkpoint inhibitors are each administered in a pharmaceutically effective amount. In some embodiments, the immune checkpoint inhibitor is an inhibitor of IDO1.

In some embodiments, the hematologic cancer is a leukemia, a lymphoma, a myeloma, a non-Hodgkin's lymphoma, a Hodgkin's lymphoma, a T-cell malignancy, or a B-cell malignancy.

In some embodiments, the hematologic cancer is a T-cell malignancy. In some embodiments, the T-cell malignancy is peripheral T-cell lymphoma not otherwise specified (PTCL-NOS), anaplastic large cell lymphoma, angioimmunoblastic lymphoma, cutaneous T-cell lymphoma, adult T-cell leukemia/lymphoma (ATLL), blastic NK-cell lymphoma, enteropathy-type T-cell lymphoma, hematosplenic gamma-delta T-cell lymphoma, lymphoblastic lymphoma, nasal NK/T-cell lymphomas, or treatment-related T-cell lymphomas.

In some embodiments, the hematologic cancer is a B-cell proliferative disorder. In some embodiments, the cancer is chronic lymphocytic leukemia (CLL), small lymphocytic lymphoma (SLL), high risk CLL, or a non-CLL/SLL lymphoma. In some embodiments, the cancer is follicular lymphoma (FL), diffuse large B-cell lymphoma (DLBCL), mantle cell lymphoma (MCL), Waldenstrom's macroglobulinemia, multiple myeloma, extranodal marginal zone B cell lymphoma, nodal marginal zone B cell lymphoma, Burkitt's lymphoma, non-Burkitt high grade B cell lymphoma, primary mediastinal B-cell lymphoma (PMBL), immunoblastic large cell lymphoma, precursor B-lymphoblastic lymphoma, B cell prolymphocytic leukemia, lymphoplasmacytic lymphoma, splenic marginal zone lymphoma, plasma cell myeloma, plasmacytoma, mediastinal (thymic) large B cell lymphoma, intravascular large B cell lymphoma, primary effusion lymphoma, or lymphomatoid granulomatosis. In some embodiments, DLBCL is further divided into subtypes: activated B-cell diffuse large B-cell lymphoma (ABC-DLBCL), germinal center diffuse large B-cell lymphoma (GCB DLBCL), and Double-Hit (DH) DLBCL. In some embodiments, ABC-DLBCL is characterized by a CD79B mutation. In some embodiments, ABC-DLBCL is characterized by a CD79A mutation. In some embodiments, the ABC-DLBCL is characterized by a mutation in MyD88, A20, or a combination thereof. In some embodiments, the cancer is acute or chronic myelogenous (or myeloid) leukemia, myelodysplastic syndrome, or acute lymphoblastic leukemia.

In some embodiments, the cancer is diffuse large B-cell lymphoma (DLBCL). In some embodiments, the cancer is activated B-cell diffuse large B-cell lymphoma (ABC-DLBCL). In some embodiments, the cancer is follicular lymphoma (FL). In some embodiments, the cancer is multiple myeloma. In some embodiments, the cancer is chronic lymphocytic leukemia (CLL). In some embodiments, the cancer is small lymphocytic lymphoma (SLL). In some embodiments, the cancer is non-CLL/SLL lymphoma. In some embodiments, the cancer is high risk CLL or high risk SLL.

In some embodiments, the hematologic cancer is a leukemia, a lymphoma, a myeloma, a non-Hodgkin's lymphoma, a Hodgkin's lymphoma, a T-cell malignancy, or a B-cell malignancy. In some embodiments, the hematologic cancer is a B-cell malignancy. In some embodiments, the B-cell malignancy is chronic lymphocytic leukemia (CLL), small lymphocytic lymphoma (SLL), high risk CLL, non-CLL/SLL lymphoma, follicular lymphoma (FL), diffuse large B-cell lymphoma (DLBCL), mantle cell lymphoma (MCL), Waldenstrom's macroglobulinemia, multiple myeloma, extranodal marginal zone B cell lymphoma, nodal marginal zone B cell lymphoma, Burkitt's lymphoma, non-Burkitt high grade B cell lymphoma, primary mediastinal B-cell lymphoma (PMBL), immunoblastic large cell lymphoma, precursor B-lymphoblastic lymphoma, B cell prolymphocytic leukemia, lymphoplasmacytic lymphoma, splenic marginal zone lymphoma, plasma cell myeloma, plasmacytoma, mediastinal (thymic) large B cell lymphoma, intravascular large B cell lymphoma, primary effusion lymphoma, or lymphomatoid granulomatosis. In some embodiments, the hematologic cancer is CLL. In some embodiments, the hematologic cancer is SLL. In some embodiments, the hematologic cancer is DLBCL. In some embodiments, the hematologic cancer is mantle cell lymphoma. In some embodiments, the hematologic cancer is FL. In some embodiments, the hematologic cancer is Waldenstrom's macroglobulinemia. In some embodiments, the hematologic cancer is multiple myeloma. In some embodiments, the hematologic cancer is Burkitt's lymphoma.

Disclosed herein, in certain embodiments, is a method for potentiating the anti-tumor activity of a checkpoint inhibitor comprising co-administering to a patient one or more TLR9 agonist and the checkpoint inhibitor. In certain embodiments of this aspect, the TLR9 agonist is administered to the cancer patient via intratumoral administration prior to the patient being administered the checkpoint inhibitor. In a preferred embodiments, the TLR9 agonist is an immunomer. In some embodiments, the immunomer is a compound selected from Table II. In some embodiments, the immune checkpoint inhibitor is an inhibitor of Programmed Death-Ligand 1 (PD-L1, also known as B7-H1, CD274), Programmed Death 1 (PD-1), CTLA-4, PD-L2 (B7-DC, CD273), LAG3, TIM3, 2B4, A2aR, B7H1, B7H3, B7H4, BTLA, CD2, CD27, CD28, CD30, CD40, CD70, CD80, CD86, CD137, CD160, CD226, CD276, DR3, GAL9, GITR, HAVCR2, HVEM, IDO1, IDO2, ICOS (inducible T cell costimulator), KIR, LAIR1, LIGHT, MARCO (macrophage receptor with collageneous structure), PS (phosphatidylserine), OX-40, SLAM, TIGHT, VISTA, VTCN1, or any combinations thereof. In some embodiments, the immune checkpoint inhibitor is an inhibitor of IDO1, CTLA4, PD-1, LAG3, PD-L1, TIM3, or combinations thereof. In some embodiments, the immune checkpoint inhibitor is an inhibitor of PD-L1. In some embodiments, the immune checkpoint inhibitor is an inhibitor of PD-1. In some embodiments, the immune checkpoint inhibitor is an inhibitor of CTLA-4. In some embodiments, the immune checkpoint inhibitor is an inhibitor of LAG3. In some embodiments, the immune checkpoint inhibitor is an inhibitor of TIM3. In some embodiments, the immune checkpoint inhibitor is an inhibitor of IDO1. In some embodiments, the one or more checkpoint inhibitors are administered by any suitable route. In some embodiments, the route of administration of the one or more checkpoint inhibitors is parenteral, mucosal delivery, oral, sublingual, transdermal, topical, inhalation, intranasal, aerosol, intratumoral, intraocular, intratracheal, intrarectal, intragastric, vaginal, by gene gun, dermal patch or in eye drop or mouthwash form. In some embodiments, the one or more TLR9 agonists and the one or more checkpoint inhibitors are each administered in a pharmaceutically effective amount. In some embodiments, the cancer is a solid tumor. In some embodiments, the cancer is a hematologic cancer.

Disclosed herein, in certain embodiments, is a method increasing or restoring the anti-tumor activity of a checkpoint inhibitor in a cancer that was previously unresponsive to, or had become resistant to, the checkpoint inhibitor, such method comprising co-administering to a patient one or more TLR9 agonist and the checkpoint inhibitor. In certain embodiments of this aspect, the TLR9 agonist is administered to the cancer patient via intratumoral administration prior to the patient being administered the checkpoint inhibitor. In some embodiments, the TLR9 agonist is an immunomer. In some embodiments, the immunomer is a compound selected from Table II. In some embodiments, the immune checkpoint inhibitor is an inhibitor of Programmed Death-Ligand 1 (PD-L1, also known as B7-H1, CD274), Programmed Death 1 (PD-1), CTLA-4, PD-L2 (B7-DC, CD273), LAG3, TIM3, 2B4, A2aR, B7H1, B7H3, B7H4, BTLA, CD2, CD27, CD28, CD30, CD40, CD70, CD80, CD86, CD137, CD160, CD226, CD276, DR3, GAL9, GITR, HAVCR2, HVEM, IDO1, IDO2, ICOS (inducible T cell costimulator), KIR, LAIR1, LIGHT, MARCO (macrophage receptor with collageneous structure), PS (phosphatidylserine), OX-40, SLAM, TIGHT, VISTA, VTCN1, or any combinations thereof. In some embodiments, the immune checkpoint inhibitor is an inhibitor of IDO1, CTLA4, PD-1, LAG3, PD-L1, TIM3, or combinations thereof. In some embodiments, the immune checkpoint inhibitor is an inhibitor of PD-L1. In some embodiments, the immune checkpoint inhibitor is an inhibitor of PD-1. In some embodiments, the immune checkpoint inhibitor is an inhibitor of CTLA-4. In some embodiments, the immune checkpoint inhibitor is an inhibitor of LAG3. In some embodiments, the immune checkpoint inhibitor is an inhibitor of TIM3. In some embodiments, the immune checkpoint inhibitor is an inhibitor of IDO1. In some embodiments, the one or more checkpoint inhibitors are administered by any suitable route. In some embodiments, the route of administration of the one or more checkpoint inhibitors is parenteral, mucosal delivery, oral, sublingual, transdermal, topical, inhalation, intranasal, aerosol, intratumoral, intraocular, intratracheal, intrarectal, intragastric, vaginal, by gene gun, dermal patch or in eye drop or mouthwash form. In some embodiments, the one or more TLR9 agonists and the one or more checkpoint inhibitors are each administered in a pharmaceutically effective amount. In some embodiments, the cancer is a solid tumor. In some embodiments, the cancer is a hematologic cancer.

In some embodiments, in any of the methods herein, the cancer is selected from the group consisting of non-Hodgkin's lymphoma, B cell lymphoma, B cell leukemia, T cell lymphoma, T cell leukemia, acute lymphoid leukemia, chronic lymphoid leukemia, Burkitt lymphoma, Hodgkin's lymphoma, hairy cell leukemia, acute myeloid leukemia, chronic myeloid leukemia, multiple myeloma, glioma, Waldenstrom's macroglobulinemia, carcinoma, melanoma, sarcoma, glioma, skin cancer, oral cavity cancer, gastrointestinal tract cancer, colon cancer, stomach cancer, pulmonary tract cancer, lung cancer, breast cancer, ovarian cancer, prostate cancer, uterine cancer, endometrial cancer, cervical cancer, urinary bladder cancer, pancreatic cancer, bone cancer, liver cancer, gall bladder cancer, kidney cancer, and testicular cancer. In some embodiment the cancer is lymphoma, colon carcinoma, or melanoma. In some embodiment the cancer is melanoma. In some embodiment the cancer is lymphoma. In some embodiment the cancer is colon carcinoma.

As used herein, the term "TLR9 agonist" generally refers to an immunostimulatory oligonucleotide compound comprising a CpG dinucleotide motif and is able to enhance or induce an immune stimulation mediated by TLR9. In some embodiments the CpG dinucleotide is selected from the group consisting of CpG, C^{∗}pG, CpG^{∗}, and C^{∗}pG^{∗}, wherein C is 2'-deoxycytidine, C^{∗} is an analog thereof, G is 2'-deoxyguanosine, and G^{∗} is an analog thereof, and p is an internucleoside linkage selected from the group consisting of phosphodiester, phosphorothioate, and phosphorodithioate. In preferred embodiments C^{∗} is selected from the group consisting of 2'-deoxythymidine, arabinocytidine, 2'-deoxythymidine, 2'-deoxy-2'-substitutedarabinocytidine, 2'-O-substitutedarabinocytidine, 2'-deoxy-5-hydroxycytidine, 2'-deoxy-N4-alkyl-cytidine, 2'-deoxy-4-thiouridine. In preferred embodiments, G^{∗} is 2' deoxy-7-deazaguanosine, 2'-deoxy-6-thioguanosine, arabinoguanosine, 2'-deoxy-2'substituted-arabinoguanosine, 2'-O-substituted-arabinoguanosine, 2'-deoxyinosine. In certain preferred embodiments, the immunostimulatory dinucleotide is selected from the group consisting of C^{∗}pG, CpG^{∗}, and C^{∗}pG^{∗}.

As used herein, an immunomer refers to a compound comprising at least two oligonucleotides linked together through their 3' ends, such that the immunomer has more than one accessible 5' end, wherein at least one of the oligonucleotides is an immunostimulatory oligonucleotide. The linkage at the 3' ends of the component oligonucleotides is independent of the other oligonucleotide linkages and may be directly via 5', 3' or 2' hydroxyl groups, or indirectly, via a non-nucleotide linker or a nucleoside, utilizing either the 2' or 3' hydroxyl positions of the nucleoside. Linkages may also utilize a functionalized sugar or nucleobase of a 3' terminal nucleotide. The term "accessible 5' end" means that the 5' end of the oligonucleotide is sufficiently available such that the factors that recognize and bind to immunomers and stimulate the immune system have access to it. Optionally, the 5'OH can be linked to a phosphate, phosphorothioate, or phosphorodithioate moiety, an aromatic or aliphatic linker, cholesterol, or another entity which does not interfere with accessibility.

As used herein, an immunostimulatory oligonucleotide is an oligodeoxyribonucleotide that comprises a CpG dinucleotide motif and is capable of enhancing or inducing a TLR9-mediated immune response. In some embodiments the CpG dinucleotide is selected from the group consisting of CpG, C^{∗}pG, CpG^{∗}, and C^{∗}pG^{∗}, wherein C is 2'-deoxycytidine, C^{∗} is an analog thereof, G is 2'-deoxyguanosine, and G^{∗} is an analog thereof, and p is an internucleoside linkage selected from the group consisting of phosphodiester, phosphorothioate, and phosphorodithioate. In preferred embodiments C^{∗} is selected from the group consisting of 2'-deoxythymidine, arabinocytidine, 2'-deoxythymidine, 2'-deoxy-2'-substitutedarabinocytidine, 2'-O-substitutedarabinocytidine, 2'-deoxy-5-hydroxycytidine, 2'-deoxy-N4-alkyl-cytidine, 2'-deoxy-4-thiouridine, In preferred embodiments, G^{∗} is 2' deoxy-7-deazaguanosine, 2'-deoxy-6-thioguanosine, arabinoguanosine, 2'-deoxy-2'substituted-arabinoguanosine, 2'-O-substituted-arabinoguanosine, 2'-deoxyinosine. In certain preferred embodiments, the immunostimulatory dinucleotide is selected from the group consisting of C^{∗}pG, CpG^{∗}, and C^{∗}pG^{∗}.

In some embodiments, the immunomer comprises two or more immunostimulatory oligonucleotides which may be the same or different. Preferably, each such immunostimulatory oligonucleotide has at least one accessible 5' end.

In some embodiments, the oligonucleotides of the immunomer each independently have from about 3 to about 35 nucleoside residues, preferably from about 4 to about 30 nucleoside residues, more preferably from about 4 to about 20 nucleoside residues. In some embodiments, the oligonucleotides have from about 5 to about 18, or from about 5 to about 14, nucleoside residues. As used herein, the term "about" implies that the exact number is not critical. Thus, the number of nucleoside residues in the oligonucleotides is not critical, and oligonucleotides having one or two fewer nucleoside residues, or from one to several additional nucleoside residues are contemplated as equivalents of each of the embodiments described above. In some embodiments, one or more of the oligonucleotides have 11 nucleotides.

In certain embodiments of the invention, the immunomers comprise two oligonucleotides covalently linked by a nucleotide linkage, or a non-nucleotide linker, at their 3'-ends or by functionalized sugar or by functionalized nucleobase via a non-nucleotide linker or a nucleotide linkage. As a non-limiting example, the linker may be attached to the 3'-hydroxyl. In such embodiments, the linker comprises a functional group, which is attached to the 3'-hydroxyl by means of a phosphate-based linkage like, for example, phosphodiester, phosphorothioate, phosphorodithioatc, methylphosphonate, or by non-phosphate-based linkages. Possible sites of conjugation for the ribonucleotide are indicated in Formula I, below, wherein B represents a heterocyclic base and wherein the arrow pointing to P indicates any attachment to phosphorous.

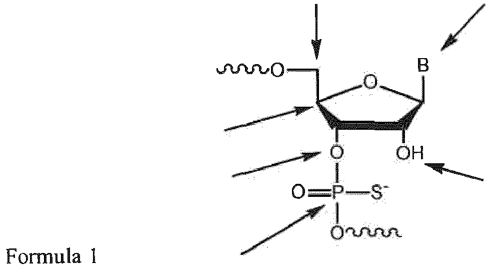

In some embodiments, the non-nucleotide linker is a small molecule, macromolecule or biomolecule, including, without limitation, polypeptides, antibodies, lipids, antigens, allergens, and oligosaccharides. In some other embodiments, the non-nucleotidic linker is a small molecule. For purposes of the invention, a small molecule is an organic moiety having a molecular weight of less than 1,000 Da. In some embodiments, the small molecule has a molecular weight of less than 750 Da.

In some embodiments, the small molecule is an aliphatic or aromatic hydrocarbon, either of which optionally can include, either in the linear chain connecting the oligoribonucleotides or appended to it, one or more functional groups including, but not limited to, hydroxy, amino, thiol, thioether, ether, amide, thioamide, ester, urea, or thiourea. The small molecule can be cyclic or acyclic. Examples of small molecule linkers include, but are not limited to, amino acids, carbohydrates, cyclodextrins, adamantane, cholesterol, haptens and antibiotics. However, for purposes of describing the non-nucleotidic linker, the term "small molecule" is not intended to include a nucleoside.

In some embodiments, the non-nucleotidic linker is an alkyl linker or amino linker. The alkyl linker may be branched or unbranched, cyclic or acyclic, substituted or unsubstituted, saturated or unsaturated, chiral, achiral or racemic mixture. The alkyl linkers can have from about 2 to about 18 carbon atoms. In some embodiments such alkyl linkers have from about 3 to about 9 carbon atoms. Some alkyl linkers include one or more functional groups including, but not limited to, hydroxy, amino, thiol, thioether, ether, amide, thioamide, ester, urea, and thioether. Such alkyl linkers can include, but are not limited to, 1,2 propanediol, 1,2,3 propanetriol, 1,3 propanediol, triethylene glycol hexaethylene glycol, polyethylene glycollinkers (e.g. [-O-CH2-CH2-]ₙ (n= 1-9)),methyl linkers, ethyl linkers, propyl linkers, butyl linkers, or hexyl linkers. In some embodiments, such alkyl linkers may include peptides or amino acids.

In some embodiments, the non-nucleotidic linker may include, but are not limited to, those listed in Table I.

**Table I: Representative Non-nucleotidic Linkers**

| | |
|---|---|
| | 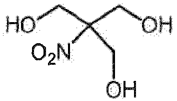 |
| 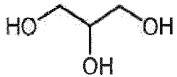 Glycerol (1,2,3-Propanetriol) | 1,1,1-Tris(hydroxymethyl)nitromethane |
| 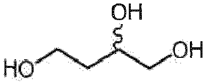 | 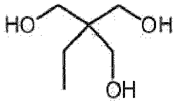 |
| 1,2,4-Butanetriol | 1,1,1-Tris(hydroxymethyl)propane |
| | 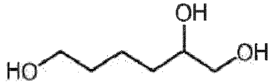 |
| 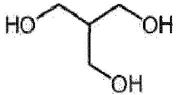 | 1,2,6-Hexanetriol |
| 2-(hydroxymethyl)-1,3-propanediol | 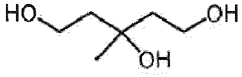 |
| 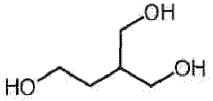 | 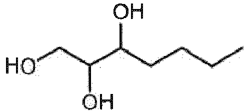 |
| 2-(hydroxymethyl)1,4-butanediol | 3-Methyl-1,3,5-pentanetriol |
| 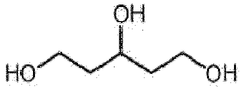 | 1,2,3-Heptanetriol |
| 1,3,5-Pentanetriol | 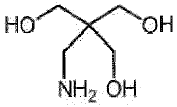 |
| 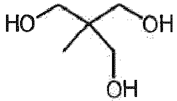 | 2-Amino-2-(hydroxymethyl)-1,3-propanediol |
| 1,1,1-Tris(hydroxymethyl)ethane | 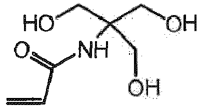 |
| | N-[Tris(hydroxymethyl)methyl]acrylamide |
| **Table I: Continued** | |
| 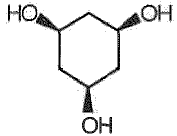 | 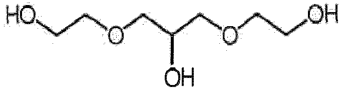 |
| cis-1,3,5-Cyclohexanetriol | 1,3-Di(hydroxyethoxy)-2-hydroxyl-propane |
| 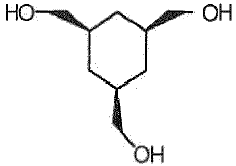 | 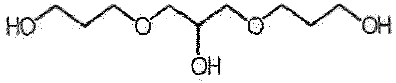 |
| cis-1,3,5-Tri(hydroxymethyl)cyclohexane | 1,3-Di(hydroxypropoxy)-2-hydroxyl-propane |
| 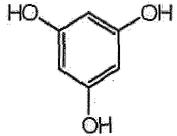 | 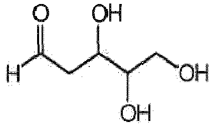 |
| 1,3,5,-Trihydroxyl-benzene | 2-Deoxy-D-ribose |
| 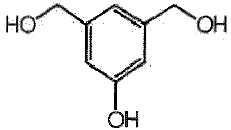 | 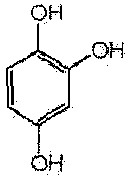 |
| 3,5,-Di(hydroxymethyl)phenol | 1,2,4,-Trihydroxyl-benzene |
| 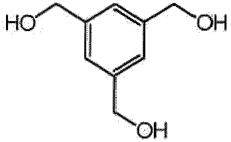 | 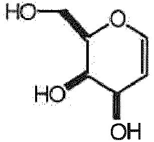 |
| 1,3,5,-Tri(hydroxymethyl(benzene | D-Galactoal |
| **Table I: Continued** | |
| 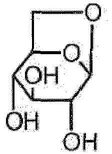 | 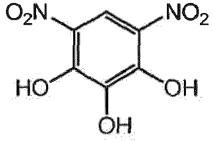 |
| 1,6-anhydro-β-D-Glucose | 4,6-Nitropyrogallol |
| 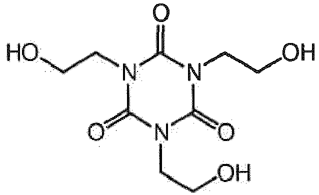 | |
| 1,3,5-Tris(2-hydroxyethyl)-Cyanuric acid | |
| 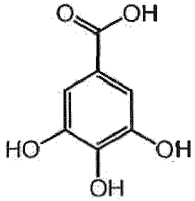 | |
| Gallic acid | |
| 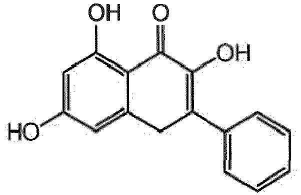 | |
| 3,5,7-Trihydroxyflavone | |
| **Table I: Continued** | |
| 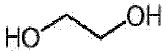 | 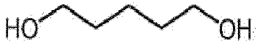 |
| Ethylene glycol | 1,5-Pentanediol |
| 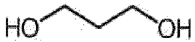 | 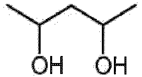 |
| 1,3-Propanediol | 2,4-Pentanediol |
| 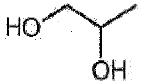 | 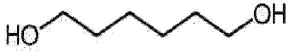 |
| 1,2-Propanediol | 1,6-Hexanediol |
| 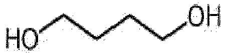 | 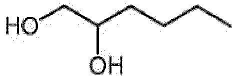 |
| 1,4-Butanediol | 1,2-Hexanediol |
| 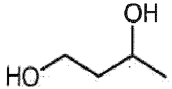 | 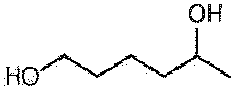 |
| 1,3-Butanediol | 1,5-Hexanediol |
| 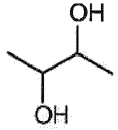 | 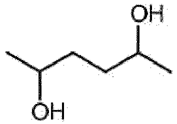 |
| 2,3-Butanediol | 2,5-Hexanediol |
| 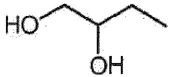 | |
| 1,4-Butanediol | |
| **Table I: Continued** | |
| 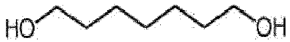 | 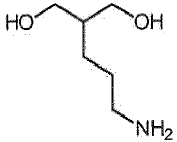 |
| 1,7-Heptanediol | |
| 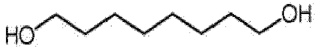 | 2-(1-Aminopropyl)-1,3-propanediol |
| 1,8-Octanediol | |
| 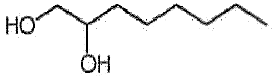 | 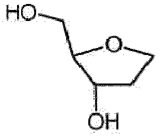 |
| 1,2-Octanediol | |
| 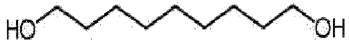 | 1,2-Dideoxyribose |
| 1,9-Nonanediol | |
| 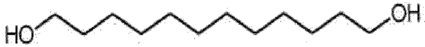 | |
| 1,12-Dodecanediol | |
| 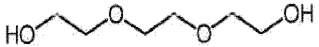 | |
| Triethylene glycol | |
| 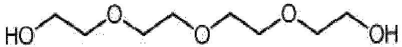 | |
| Tetraethylene glycol | |
| 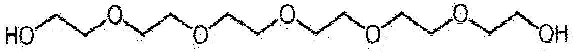 | |
| Hexaethylene glycol | |
| 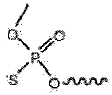 | 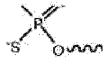 |
| Glycerol linker | Pentanetriol linker |
| 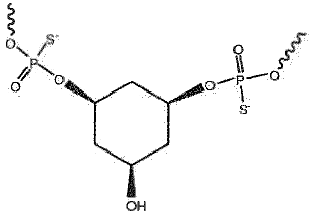 | 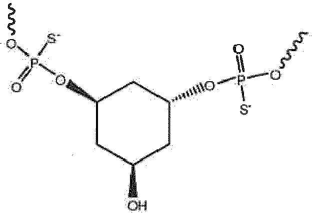 |
| Cis, cis-cyclohexanetriol linker | Cis, trans-cyclohexanetriol linker |
| 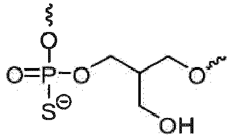 | 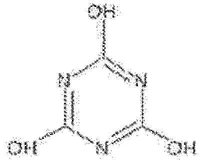 |
| 1,3,4- Isobutanetriol | Cyanuric Acid |

The oligonucleotides of the immunomer can, independently, include naturally occurring nucleosides, modified nucleosides, or mixtures thereof. The oligonucleotides of the immunomer can also, independently, be selected from hybrid and chimeric oligonucleotides. A "chimeric oligonucleotide" is an oligonucleotide having more than one type of internucleoside linkage. One preferred example of such a chimeric oligonucleotide is a chimeric oligonucleotide comprising a phosphorothioate, phosphodiester or phosphorodithioate region and non-ionic linkages such as alkylphosphonate or alkylphosphonothioate linkages (see *e.g.,* Pederson *et al.* U.S. Patent Nos. 5,635,377 and 5,366,878).

A "hybrid oligonucleotide" is an oligonucleotide having more than one type of nucleoside. One preferred example of such a hybrid oligonucleotide comprises a ribonucleotide or 2'-substituted ribonucleotide region, and a deoxyribonucleotide region (see, e.g., Metelev and Agrawal, U.S. Patent No. 5,652,355, 6,346,614 and 6,143,881).

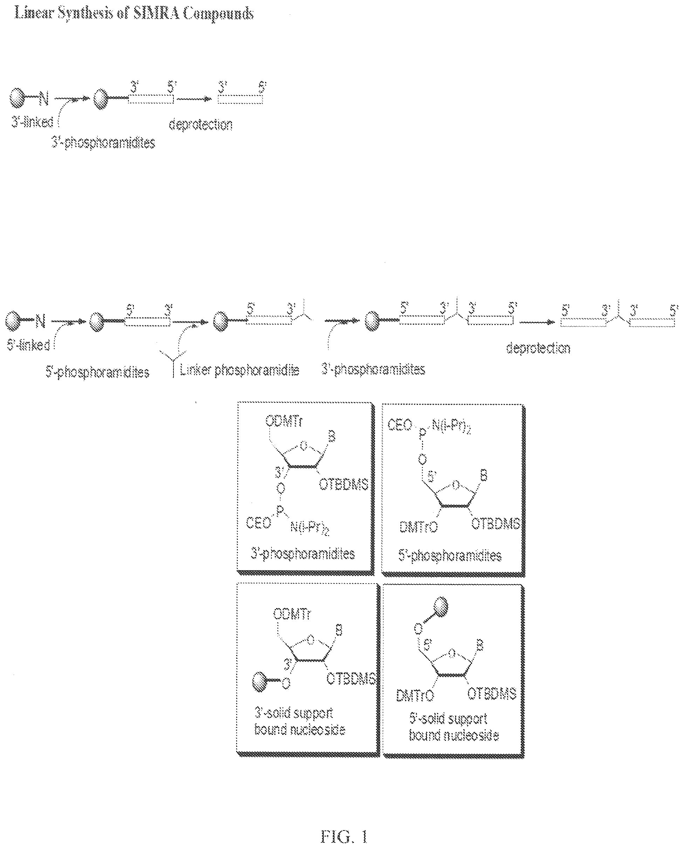

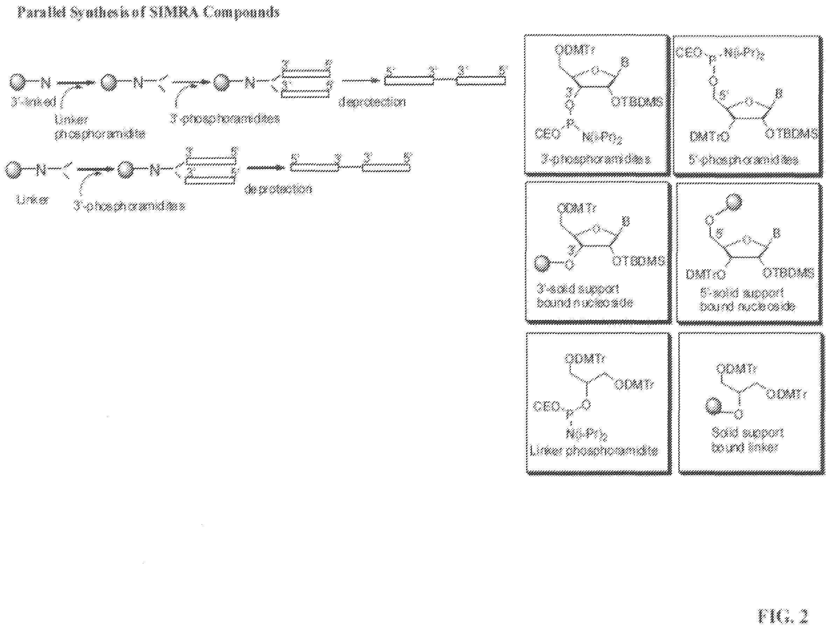

The immunomers may conveniently be synthesized using an automated synthesizer and phosphoramidite approach as schematically depicted in Figures 1 and 2, and further described in the Examples. In some embodiments, the immunomers are synthesized by a linear synthesis approach (see Figure 1). As used herein, the term "linear synthesis" refers to a synthesis that starts at one end of the immunomer and progresses linearly to the other end. Linear synthesis permits incorporation of either identical or un-identical (in terms of length, base composition and/or chemical modifications incorporated) monomeric units into the immunomers.

One alternative mode of synthesis is, for example, "parallel synthesis", in which synthesis proceeds outward from a central linker moiety (see Figure 2). A solid support attached linker can be used for parallel synthesis, as is described in U.S. Patent No. 5,912,332. Alternatively, a universal solid support (such as phosphate attached controlled pore glass support can be used.

Parallel synthesis of immunomers has several advantages over linear synthesis: (1) parallel synthesis permits the incorporation of identical monomeric units; (2) unlike in linear synthesis, both (or all) the monomeric units are synthesized at the same time, thereby the number of synthetic steps and the time required for the synthesis is the same as that of a monomeric unit; and (3) the reduction in synthetic steps improves purity and yield of the final immunomer product.

At the end of the synthesis by either linear synthesis or parallel synthesis protocols, the immunomers may conveniently be deprotected with concentrated ammonia solution or as recommended by the phosphoramidite supplier, if a modified nucleoside is incorporated. The product immunomer is preferably purified by reversed phase HPLC, detritylated, desalted and dialyzed.

Table II shows representative immunomers. All internucleotide linkages are phosphorothioate unless otherwise noted.

**TABLE II**

| IMO # | Sequence (SEQ ID NO:) |
|---|---|
| 1 | 5'-TCTGACG₁TTCT-X-TCTTG₁CAGTCT-5' (5'-SEQ ID NO:1-X-SEQ ID NO:1-5') |
| 2 | 5'-TCTGTCG₁TTCT-X-TCTTG₁CTGTCT-5' (5'-SEQ ID NO:2-X-SEQ ID NO:2-5') |
| 3 | 5-TCG₁TCG₁TTCTG-X-GTCTTG₁CTG₁CT-5' (5'-SEQ ID NO:3-X-SEQ ID NO:3-5') |
| 4 | S-TCG₁AACG₁TTCG₁-X-G₁CTTG₁CAAG₁CT-5' (5'-SEQ ID NO:4-X-SEQ ID NO:4-5') |
| 5 | 5-CTGTCoG₂TTCTC-X-CTCTTG₂oCTGTC-5' (5'-SEQ ID NO:5-X-SEQ ID NO:5-5') |
| 6 | 5-CTGTCG₂TTCTCo-X-oCTCTTG₂CTGTC-5' (5'-SEQ ID NO:6-X-SEQ ID NO:6-5') |
| 7 | 5'-TCG₁AACG₁TTCG₁-X-TCTTG₂CTGTCT-5' (5'-SEQ ID NO:4-X-SEQ ID NO:21-5') |
| 8 | 5-TCG₁AACG₁TTCG₁-Y-GACAG₁CTGTCT-5' (5'-SEQ ID NO:4-X-SEQ ID NO:22-5') |
| 9 | 5-CAGTCG₂TTCAG-X-GACTTG₂CTGAC-5' (5'-SEQ ID NO:7-X-SEQ ID NO:7-5') |
| 10 | 5-CAGTCG₁TTCAG-X-GACTTG₁CTGAC-5' (5'-SEQ ID NO:8-X-SEQ ID NO:8-5') |
| 11 | 5'-TCG₁AACG₁TTCoG-Z-GoCTTG₁CAAG₁CT-5' (5'-SEQ ID NO:9-X-SEQ ID NO:9-5') |
| 12 | 5'-TCG₁AACG₁TTCG₁-Y₂-TC1TG₁CTGTCTTG₁CT-5' (5'-SEQ ID NO:4-X-SEQ ID NO:23-5') |
| 13 | 5'-TCG₁AACG₁TTCG₁-Y₂-TCTTG₁CTGUCT-5' (5'-SEQ ID NO:4-X-SEQ ID NO:24-5') |
| 14 | 5'-TCG₁AACG₁ToTCoG-m-GoCToTG₁CAAG₁CT-5' (5'-SEQ ID NO:10-X-SEQ ID NO:10-5') |
| 15 | 5'-TCG₁ACG₁TCoG-Y₃-GACTTG₂CTGAC-5' (5'-SEQ ID NO:9-X-SEQ ID NO:7-5') |
| 16 | 5'-TCG₁AACG₁TCGG₁-Y₄-TGTTG₁CTGTCTTG₁CT-5' (5'-SEQ ID NO:4-X-SEQ ID NO:25-5') |
| 17 | 5'-TCG₂TCG₂TTU₁Y-M-YU₁TTG₂CTG₂CT-5' (5'-SEQ ID NO:11-X-SEQ ID NO:11-5') |
| 18 | 5'-CAGTCG₂TTCAG-Y₃-TCTTG₁CTGTCT-5' (5'-SEQ ID NO:7-X-SEQ ID NO:2-5') |
| 19 | 5'-TCG₁TACG₁TACG₁-X-G₁CATG₁CATG₁CT-5' (5'-SEQ ID NO:12-X-SEQ ID NO:12-5') |
| 20 | 5'-TCG₁AACG₁TTCG-Z-GCTTG₁CAAG₁CT-5' (5'-SEQ ID NO:13-X-SEQ ID NO:13-5') |
| 21 | 5'-TCG₁AACG₁TTCoG-Y₃-CTTG₂CTGACTTG₁CT-5' (5'-SEQ ID NO:14-X-SEQ ID NO:26-5') |
| 22 | 5'-TCG₁AACG₁oTTCG₁-X₂-G₁CTToG₁CAAG₁CT-5' (5'-SEQ ID NO:15-X-SEQ ID NO:15-5') |
| 23 | 5'-TCG₁AACG₁TTCG₁-Y₄-CATTG₁CTGTCTTG₁CT (5'-SEQ ID NO:4-X-SEQ ID NO:27-5') |
| 24 | 5'-TCG₁AACG₁TTCG₁-m-G₁CTTG₁CAAG₁CT-5' (5'-SEQ ID NO:4-X-SEQ ID NO:4-5') |
| 25 | 5'-TCoG₁oAACoG₁TTCoG₁o-X₂-oG₁oCTTG₁oCAAoG₁oCT-5' (5'-SEQ ID NO:16-X-SEQ ID NO:16-5') |
| 26 | 5'-ToCG₁oAACoG₁TTCoG₁o-X₂-oG₁oCTTG₁oCAAoG₁CoT-5' (5'-SEQ ID NO:17-X-SEQ ID NO:17-5') |
| 27 | 5'-TCoG1oAACoG₁TTCoG₁o-m-oG₁oCTTG₁oCAAoG₁oCT-5' (5'-SEQ ID NO:16-X-SEQ ID NO:16-5') |
| 28 | 5'-TCoG₂oAACoG₂TTCoG₂o-X₂-oG₂oCTTG₂oCAAoG₂oCT-5' (5'-SEQ ID NO:18-X-SEQ ID NO:18-5') |
| 29 | 5'-TCoG₁oAACoG₁TTCoGo-Z-oGoCTTG₁oCAAoG₁oCT-5' (5'-SEQ ID NO:19-X-SEQ ID NO:19-5') |
| 30 | 5'-ToCG₁oAACoG₁TTCoGo-Z-oGoCTTG₁oCAAoG₁CoT-5' (5'-SEQ ID NO:20-X-SEQ ID NO:20-5') |

G₁ is 2'-deoxy-7-deazaguanosine; G₂ is 2'-deoxy-arabinoguanosine; G/C/U is 2'-O-methylribonucleotides; U₁ is 2'-deoxy-U; o is phosphodiester linkage; X is a glycerol linker; X₂ = Isobutanetriol linker, Y is C3-linker; m is cis,trans-1,3,5-cyclohexanetriol linker; Y₂ is 1,3-propanediol linker; Y₃ is 1,4-butanediol linker; Y₄ is 1,5-pentandiol linker; Z is 1,3,5-pentanetriol linker; M is cis,cis-1,3,5-cyclohexanetriol linker.

Immune checkpoints refer to inhibitory pathways in the immune system that are responsible for maintaining self-tolerance and modulating the degree of immune system response to minimize peripheral tissue damage. The induction of an immune response, whether through infection by a pathogen (e.g., bacteria, virus, or fungus) or through the administration of a synthetic immune agonist (e.g., a TLR9 agonist) leads to the upregulation of immune checkpoints. However, it has been shown that tumor cells can also activate immune system checkpoints to decrease the effectiveness of immune response against tumor tissues. Exemplary checkpoint molecules include, but are not limited to, cytotoxic T-lymphocyte antigen 4 (CTLA4, also known as CD152), programmed cell death protein 1 (PD-1, also known as CD279), programmed cell death 1 ligand 1 (PD-L1, also known as CD274), lymphocyte activation gene-3 (LAG-3; CD223), B7-H3, B7-H4, killer immunoglobulin receptor (KIR), Tumor necrosis factor receptor superfamily, member 4 (TNFRSF4, also known as OX40 and CD134) and its ligand OX40L (CD252), indoleamine 2,3-dioxygenase 1 (IDO-1), indoleamine 2,3-dioxygenase 2 (IDO-2), carcinoembryonic antigen-related cell adhesion molecule 1 (CEACAM1), B and T lymphocyte attenuator (BTLA; also known as CD272), and T-cell membrane protein 3 (TIM3). In preferred embodiments, the checkpoint is CTLA4, IDO-1, PD-L1, or PD-1. In preferred embodiments, the checkpoint is CTLA4. In preferred embodiments, the checkpoint is PD-L1. In preferred embodiments, the checkpoint is IDO-1. In preferred embodiments, the checkpoint is PD-1.

Any suitable immune checkpoint inhibitor is contemplated for use with the methods disclosed herein. "Immune checkpoint inhibitors," as used herein refer to any modulator that inhibits the activity of the immune checkpoint molecule. Immune checkpoint inhibitors can include, but are not limited to, immune checkpoint molecule binding proteins, small molecule inhibitors, antibodies, antibody-derivatives (including Fab fragments and scFvs), antibody-drug conjugates, antisense oligonucleotides, siRNA, aptamers, peptides and peptide mimetics. Inhibitory nucleic acids that decrease the expression and/or activity of immune checkpoint molecules can also be used in the methods disclosed herein. One embodiment is a small inhibitory RNA (siRNA) for interference or inhibition of expression of a target gene. Nucleic acid sequences encoding PD-1, PD-L1 and PD-L2 are disclosed in GENBANK^{®} Accession Nos. NM 005018, AF344424, NP_079515, and NP_054862.

In one embodiment, the immune checkpoint inhibitor reduces the expression or activity of one or more immune checkpoint proteins. In another embodiment, the immune checkpoint inhibitor reduces the interaction between one or more immune checkpoint proteins and their ligands.

In some embodiments, the immune checkpoint inhibitor is an inhibitor of PD-L1. In some embodiments, the immune checkpoint inhibitor is an antibody against PD-L1. In some embodiments, the immune checkpoint inhibitor is a monoclonal antibody against PD-L1. In other or additional embodiments, the immune checkpoint inhibitor is a human or humanized antibody against PD-L1. In one embodiment, the immune checkpoint inhibitor reduces the expression or activity of one or more immune checkpoint proteins, such as PD-L1. In another embodiment, the immune checkpoint inhibitor reduces the interaction between PD-1 and PD-L1. Exemplary immune checkpoint inhibitors include antibodies (e.g., an anti-PD-L1 antibody), RNAi molecules (e.g., anti-PD-L1 RNAi), antisense molecules (e.g., an anti-PD-L1 antisense RNA), dominant negative proteins (e.g., a dominant negative PD-L1 protein), and small molecule inhibitors. Antibodies include monoclonal antibodies, humanized antibodies, deimmunized antibodies, and Ig fusion proteins. An exemplary anti-PD-L1 antibody includes clone EH12. Exemplary antibodies against PD-L1 include: Genentech's MPDL3280A (RG7446); Anti-mouse PD-L1 antibody Clone 10F.9G2 (Cat #BE0101) from BioXcell; anti-PD-L1 monoclonal antibody MDX-1105 (BMS-936559) and BMS-935559 from Bristol-Meyer's Squibb; MSB0010718C; mouse anti-PD-L1 Clone 29E.2A3; and AstraZeneca's MEDI4736. In some embodiments, the anti-PD-L1 antibody is an anti-PD-L 1 antibody disclosed in any of the following patent publications (herein incorporated by reference): WO2013079174; CN101104640; WO2010036959; WO2013056716; WO2007005874; WO2010089411; WO2010077634; WO2004004771; WO2006133396; WO201309906; US 20140294898; WO2013181634 or WO2012145493.

In some embodiments, the PD-L1 inhibitor is a nucleic acid inhibitor of PD-L1 expression. In some embodiments, the PD-L1 inhibitor is disclosed in one of the following patent publications (incorporated herein by reference): WO2011127180 or WO2011000841. In some embodiments, the PD-L1 inhibitor is rapamycin.

In some embodiments, the immune checkpoint inhibitor is an inhibitor of PD-L2. In some embodiments, the immune checkpoint inhibitor is an antibody against PD-L2. In some embodiments, the immune checkpoint inhibitor is a monoclonal antibody against PD-L2. In other or additional embodiments, the immune checkpoint inhibitor is a human or humanized antibody against PD-L2. In some embodiments, the immune checkpoint inhibitor reduces the expression or activity of one or more immune checkpoint proteins, such as PD-L2. In other embodiments, the immune checkpoint inhibitor reduces the interaction between PD-1 and PD-L2. Exemplary immune checkpoint inhibitors include antibodies (e.g., an anti-PD-L2 antibody), RNAi molecules (e.g., an anti-PD-L2 RNAi), antisense molecules (e.g., an anti-PD-L2 antisense RNA), dominant negative proteins (e.g., a dominant negative PD-L2 protein), and small molecule inhibitors. Antibodies include monoclonal antibodies, humanized antibodies, deimmunized antibodies, and Ig fusion proteins.

In some embodiments, the PD-L2 inhibitor is GlaxoSmithKline's AMP-224 (Amplimmune). In some embodiments, the PD-L2 inhibitor is rHIgM12B7.

In some embodiments, the immune checkpoint inhibitor is an inhibitor of PD-L1. In some embodiments, the immune checkpoint inhibitor is an antibody against PD-1. In some embodiments, the immune checkpoint inhibitor is a monoclonal antibody against PD-1. In other or additional embodiments, the immune checkpoint inhibitor is a human or humanized antibody against PD-1. For example, the inhibitors of PD-1 biological activity (or its ligands) disclosed in U.S. Pat. Nos. 7,029,674; 6,808,710; or U.S. Patent Application Nos: 20050250106 and 20050159351 can be used in the methods provided herein. Exemplary antibodies against PD-1 include: Anti-mouse PD-1 antibody Clone J43 (Cat #BE0033-2) from BioXcell; Anti-mouse PD-1 antibody Clone RMP1-14 (Cat #BE0146) from BioXcell; mouse anti-PD-1 antibody Clone EH12; Merck's MK-3475 anti-mouse PD-1 antibody (Keytruda, pembrolizumab, lambrolizumab); and AnaptysBio's anti-PD-1 antibody, known as ANB011; antibody MDX-1 106 (ONO-4538); Bristol-Myers Squibb's human IgG4 monoclonal antibody nivolumab (Opdivo.RTM., BMS-936558, MDX1106); AstraZeneca's AMP-514, and AMP-224; and Pidilizumab (CT-011), CureTech Ltd.

Additional exemplary anti-PD-1 antibodies and methods for their use are described by Goldberg et al, Blood 1 10(1): 186-192 (2007), Thompson et al, Clin. Cancer Res. 13(6): 1757-1761 (2007), and Korman et al, International Application No. PCT/JP2006/309606 (publication no. WO 2006/121168 A1), each of which are expressly incorporated by reference herein. In some embodiments, the anti-PD-1 antibody is an anti-PD-1 antibody disclosed in any of the following patent publications (herein incorporated by reference): WO014557; WO2011110604; WO2008156712; US2012023752; WO2011110621; WO2004072286; WO2004056875; WO20100036959; WO2010029434; WO201213548; WO2002078731; WO2012145493; WO2010089411; WO2001014557; WO2013022091; WO2013019906; WO2003011911; US20140294898; and WO2010001617.

In some embodiments, the PD-1 inhibitor is a PD-1 binding protein as disclosed in WO200914335 (herein incorporated by reference).

In some embodiments, the PD-1 inhibitor is a peptidomimetic inhibitor of PD-1 as disclosed in WO2013132317 (herein incorporated by reference).

In some embodiments, the PD-1 inhibitor is an anti-mouse PD-1 mAb: clone J43, BioXCell (West Lebanon, NH).

In some embodiments, the PD-1 inhibitor is a PD-L1 protein, a PD-L2 protein, or fragments, as well as antibody MDX 1 106 (ONO 4538) tested in clinical studies for the treatment of certain malignancies (Brahmer et al., J Clin Oncol. 2010 28(19): 3167-75, Epub 2010 Jun. 1). Other blocking antibodies may be readily identified and prepared by the skilled person based on the known domain of interaction between PD-1 and PD-L1/PD-L2, as discussed above. For example, a peptide corresponding to the IgV region of PD-1 or PD-L1/PD-L2 (or to a portion of this region) could be used as an antigen to develop blocking antibodies using methods well known in the art.

In some embodiments, the immune checkpoint inhibitor is an inhibitor of IDO1. In some embodiments, the immune checkpoint inhibitor is a small molecule against IDO1. Exemplary small molecules against IDO1 include: Incyte's INCB024360 ( 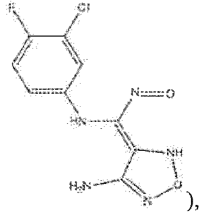 NSC-721782 (also known as 1-methyl-D-tryptophan), and Bristol Meyers Squibb's F001287.

In some embodiments, the immune checkpoint inhibitor is an inhibitor of CTLA-4. In some embodiments, the immune checkpoint inhibitor is an antibody against CTLA-4. In some embodiments, the immune checkpoint inhibitor is a monoclonal antibody against CTLA-4. In other or additional embodiments, the immune checkpoint inhibitor is a human or humanized antibody against CTLA-4. In one embodiment, the anti-CTLA-4 antibody blocks the binding of CTLA-4 to CD80 (B7-1) and/or CD86 (B7-2) expressed on antigen presenting cells. Exemplary antibodies against CTLA-4 include: Bristol Meyers Squibb's anti-CTLA-4 antibody ipilimumab (also known as Yervoy.RTM., MDX-010, BMS-734016 and MDX-101); anti-CTLA4 Antibody, clone 9H10 from Millipore; Pfizer's tremelimumab (CP-675,206, ticilimumab); and anti-CTLA4 antibody clone BNI3 from Abcam.

In some embodiments, the anti-CTLA-4 antibody is an anti-CTLA-4 antibody disclosed in any of the following patent publications (herein incorporated by reference):WO 2001014424; WO 2004035607; US2005/0201994; EP 1212422 B1; WO2003086459; WO2012120125; WO2000037504; WO2009100140; WO200609649; WO2005092380; WO2007123737; WO2006029219; WO20100979597; WO200612168; and WO1997020574. Additional CTLA-4 antibodies are described in U.S. Pat. Nos. 5,811,097, 5,855,887, 6,051,227, and 6,984,720; in PCT Publication Nos. WO 01/14424 and WO 00/37504; and in U.S. Publication Nos. 2002/0039581 and 2002/086014; and/or U.S. Pat. Nos. 5,977,318, 6,682,736, 7, 109,003, and 7,132,281, incorporated herein by reference). In some embodiments, the anti-CTLA-4 antibody is an, for example, those disclosed in: WO 98/42752; U.S. Pat. Nos. 6,682,736 and 6,207,156; Hurwitz et al, Proc. Natl. Acad. Sci. USA, 95(17): 10067-10071 (1998); Camacho et al, J. Clin. Oncol., 22(145): Abstract No. 2505 (2004) (antibody CP-675206); Mokyr et al, Cancer Res., 58:5301-5304 (1998) (incorporated herein by reference).

In some embodiments, the CTLA-4 inhibitor is a CTLA-4 ligand as disclosed in WO1996040915.

In some embodiments, the CTLA-4 inhibitor is a nucleic acid inhibitor of CTLA-4 expression. For example, anti-CTLA4 RNAi molecules may take the form of the molecules described by Mello and Fire in PCT Publication Nos. WO 1999/032619 and WO 2001/029058; U.S. Publication Nos. 2003/0051263, 2003/0055020, 2003/0056235, 2004/265839, 2005/0100913, 2006/0024798, 2008/0050342, 2008/0081373,2008/0248576, and 2008/055443; and/or U.S. Pat. Nos. 6,506,559, 7,282,564, 7,538,095, and 7,560,438 (incorporated herein by reference). In some instances, the anti-CTLA4 RNAi molecules take the form of double stranded RNAi molecules described by Tuschl in European Patent No. EP 1309726 (incorporated herein by reference). In some instances, the anti-CTLA4 RNAi molecules take the form of double stranded RNAi molecules described by Tuschl in U.S. Pat. Nos. 7,056,704 and 7,078,196 (incorporated herein by reference). In some embodiments, the CTLA4 inhibitor is an aptamer described in PCT Publication No. WO2004081021.

Additionally, the anti-CTLA4 RNAi molecules of the present invention may take the form be RNA molecules described by Crooke in U.S. Pat. Nos. 5,898,031, 6,107,094, 7,432,249, and 7,432,250, and European Application No. EP 0928290 (incorporated herein by reference).

In some embodiments, the immune checkpoint inhibitor is an inhibitor of LAG3 (CD223). In some embodiments, the immune checkpoint inhibitor is an antibody against LAG3. In some embodiments, the immune checkpoint inhibitor is a monoclonal antibody against LAG3. In other or additional embodiments, the immune checkpoint inhibitor is a human or humanized antibody against LAG3. In additional embodiments, an antibody against LAG3 blocks the interaction of LAG3 with major histocompatibility complex (MHC) class II molecules. Exemplary antibodies against LAG3 include: anti-Lag-3 antibody clone eBioC9B7W (C9B7W) from eBioscience; anti-Lag3 antibody LS-B2237 from LifeSpan Biosciences; IMP321 (ImmuFact) from Immutep; anti-Lag3 antibody BMS-986016; and the LAG-3 chimeric antibody A9H12. In some embodiments, the anti-LAG3 antibody is an anti-LAG3 antibody disclosed in any of the following patent publications (herein incorporated by reference): WO2010019570; WO2008132601; or WO2004078928.

In some embodiments, the immune checkpoint inhibitor is an antibody against TIM3 (also known as HAVCR2). In some embodiments, the immune checkpoint inhibitor is a monoclonal antibody against TIM3. In other or additional embodiments, the immune checkpoint inhibitor is a human or humanized antibody against TIM3. In additional embodiments, an antibody against TIM3 blocks the interaction of TIM3 with galectin-9 (Gal9). In some embodiments, the anti-TIM3 antibody is an anti-TIM3 antibody disclosed in any of the following patent publications (herein incorporated by reference): WO2013006490; WO201155607; WO2011159877; or WO200117057. In another embodiment, a TIM3 inhibitor is a TIM3 inhibitor disclosed in WO2009052623.

In some embodiments, the immune checkpoint inhibitor is an antibody against B7-H3. In one embodiment, the immune checkpoint inhibitor is MGA271.

In some embodiments, the immune checkpoint inhibitor is an antibody against MR. In one embodiment, the immune checkpoint inhibitor is Lirilumab (IPH2101). In some embodiments, an antibody against MR blocks the interaction of KIR with HLA.

In some embodiments, the immune checkpoint inhibitor is an antibody against CD137 (also known as 4-1BB or TNFRSF9). In one embodiment, the immune checkpoint inhibitor is urelumab (BMS-663513, Bristol-Myers Squibb), PF-05082566 (anti-4-1BB, PF-2566, Pfizer), or XmAb-5592 (Xencor). In one embodiment, an anti-CD 137 antibody is an antibody disclosed in U.S. Published Application No. US 2005/0095244; an antibody disclosed in issued U.S. Pat. No. 7,288,638 (such as 20H4.9-IgG4 [10C7 or BMS-663513] or 20H4.9-IgG1 [BMS-663031]); an antibody disclosed in issued U.S. Pat. No. 6,887,673 [4E9 or BMS-554271]; an antibody disclosed in issued U.S. Pat. No. 7,214,493; an antibody disclosed in issued U.S. Pat. No. 6,303,121; an antibody disclosed in issued U.S. Pat. No. 6,569,997; an antibody disclosed in issued U.S. Pat. No. 6,905,685; an antibody disclosed in issued U.S. Pat. No. 6,355,476; an antibody disclosed in issued U.S. Pat. No. 6,362,325 [1D8 or BMS-469492; 3H3 or BMS-469497; or 3E1]; an antibody disclosed in issued U.S. Pat. No. 6,974,863 (such as 53A2); or an antibody disclosed in issued U.S. Pat. No. 6,210,669 (such as 1D8, 3B8, or 3E1). In a further embodiment, the immune checkpoint inhibitor is one disclosed in WO 2014036412. In another embodiment, an antibody against CD137 blocks the interaction of CD137 with CD137L.

In some embodiments, the immune checkpoint inhibitor is an antibody against PS. In one embodiment, the immune checkpoint inhibitor is Bavituximab.

In some embodiments, the immune checkpoint inhibitor is an antibody against CD52. In one embodiment, the immune checkpoint inhibitor is alemtuzumab.

In some embodiments, the immune checkpoint inhibitor is an antibody against CD30. In one embodiment, the immune checkpoint inhibitor is brentuximab vedotin. In another embodiment, an antibody against CD30 blocks the interaction of CD30 with CD30L.

In some embodiments, the immune checkpoint inhibitor is an antibody against CD33. In one embodiment, the immune checkpoint inhibitor is gemtuzumab ozogamicin.

In some embodiments, the immune checkpoint inhibitor is an antibody against CD20. In one embodiment, the immune checkpoint inhibitor is ibritumomab tiuxetan. In another embodiment, the immune checkpoint inhibitor is ofatumumab. In another embodiment, the immune checkpoint inhibitor is rituximab. In another embodiment, the immune checkpoint inhibitor is tositumomab.

In some embodiments, the immune checkpoint inhibitor is an antibody against CD27 (also known as TNFRSF7). In one embodiment, the immune checkpoint inhibitor is CDX-1127 (Celldex Therapeutics). In another embodiment, an antibody against CD27 blocks the interaction of CD27 with CD70.

In some embodiments, the immune checkpoint inhibitor is an antibody against OX40 (also known as TNFRSF4 or CD 134). In one embodiment, the immune checkpoint inhibitor is anti-OX40 mouse IgG. In another embodiment, an antibody against OX40 blocks the interaction of OX40 with OX40L.

In some embodiments, the immune checkpoint inhibitor is an antibody against glucocorticoid-induced tumor necrosis factor receptor (GITR). In one embodiment, the immune checkpoint inhibitor is TRX518 (GITR, Inc.). In another embodiment, an antibody against GITR blocks the interaction of GITR with GITRL.

In some embodiments, the immune checkpoint inhibitor is an antibody against inducible T-cell COStimulator (ICOS, also known as CD278). In one embodiment, the immune checkpoint inhibitor is MEDI570 (MedImmune, LLC) or AMG557 (Amgen). In another embodiment, an antibody against ICOS blocks the interaction of ICOS with ICOSL and/or B7-H2.

In some embodiments, the immune checkpoint inhibitor is an inhibitor against BTLA (CD272), CD160, 2B4, LAIR1, TIGHT, LIGHT, DR3, CD226, CD2, or SLAM. As described elsewhere herein, an immune checkpoint inhibitor can be one or more binding proteins, antibodies (or fragments or variants thereof) that bind to immune checkpoint molecules, nucleic acids that downregulate expression of the immune checkpoint molecules, or any other molecules that bind to immune checkpoint molecules (i.e. small organic molecules, peptidomimetics, aptamers, etc.). In some instances, an inhibitor of BTLA (CD272) is HVEM. In some instances, an inhibitor of CD160 is HVEM. In some cases, an inhibitor of 2B4 is CD48. In some instances, an inhibitor of LAIR1 is collagen. In some instances, an inhibitor of TIGHT is CD112, CD113, or CD155. In some instances, an inhibitor of CD28 is CD80 or CD86. In some instances, an inhibitor of LIGHT is HVEM. In some instances, an inhibitor of DR3 is TL1A. In some instances, an inhibitor of CD226 is CD155 or CD112. In some cases, an inhibitor of CD2 is CD48 or CD58. In some cases, SLAM is self inhibitory and an inhibitor of SLAM is SLAM.

In preferred embodiments, the checkpoint inhibitor is an inhibitor of CTLA4, PD-L1, IDO1 or PD-1 or combinations thereof. In preferred embodiments, the checkpoint inhibitor is an inhibitor of CTLA4. In preferred embodiments, the checkpoint inhibitor is an inhibitor of IDO-1. In preferred embodiments, the checkpoint inhibitor is an inhibitor of PD-1.

In any of the methods according to the invention, the one or more TLR9 agonist and/or the one or more checkpoint inhibitor is included in the pharmaceutically acceptable carrier or diluent in an amount sufficient to deliver to a patient a pharmaceutically effective amount.

In any of the methods according to the invention, co-administration of the one or more TLR9 agonist and/or one or more checkpoint inhibitors can be carried out using known procedures at dosages and for periods of time effective to reduce symptoms or surrogate markers of the disease. It may be desirable to administer simultaneously, or sequentially a pharmaceutically effective amount of one or more of the therapeutic compositions of the invention to an individual as a single treatment episode.

In any of the methods according to the invention, the one or more TLR9 agonist and/or one or more checkpoint inhibitors can be further co-administered or administered in combination with any other agent useful for preventing or treating the disease or condition that does not abolish the effect of the TLR9 agonist or checkpoint inhibitor. In any of the methods according to the invention, the agent useful for preventing or treating the disease or condition includes, but is not limited to, vaccines, antigens, antibodies, cytotoxic agents, chemotherapeutic agents, allergens, antibiotics, antisense oligonucleotides, TLR agonists, kinase inhibitors, peptides, proteins, gene therapy vectors, DNA vaccines and/or adjuvants to enhance the specificity or magnitude of the immune response, or co-stimulatory molecules such as cytokines, chemokines, protein ligands, trans-activating factors, peptides and peptides comprising modified amino acids. For example, in the prevention and/or treatment of cancer, it is contemplated that a chemotherapeutic agent or a monoclonal antibody may be co-administered or administered in combination with the TLR9 agonist or checkpoint inhibitor. Preferred chemotherapeutic agents include, without limitation Gemcitabine methotrexate, vincristine, adriamycin, cisplatin, non-sugar containing chloroethylnitrosoureas, 5-fluorouracil, mitomycin C, bleomycin, doxorubicin, dacarbazine, TAXOL^{®}, fragyline, Meglamine GLA, valrubicin, carmustaine and poliferposan, MMI270, BAY 12-9566, RAS famesyl transferase inhibitor, famesyl transferase inhibitor, MMP, MTA/LY231514, LY264618/Lometexol, Glamolec, CI-994, TNP-470, Hycamtin/Topotecan, PKC412, Valspodar/PSC833, NOVANTRONE^{®}/Mitroxantrone, Metaret/Suramin, Batimastat, E7070, BCH-4556, CS-682, 9-AC, AG3340, AG3433, Incel/VX-710, VX-853, ZD0101, ISI641, ODN 698, TA 2516/Marmistat, BB2516/Marmistat, CDP 845, D2163, PD183805, DX8951f, Lemonal DP 2202, FK 317, imatinib mesylate/GLEEVEC^{®}, Picibanil/OK-432, AD 32/Valrubicin, METASTRON^{®}/strontium derivative, Temodal/Temozolomide, Evacet/liposomal doxorubicin, Yewtaxan/Placlitaxel, TAXOL^{®}/Paclitaxel, Xeload/Capecitabine, Furtulon/Doxifluridine, Cyclopax/oral paclitaxel, Oral Taxoid, SPU-077/Cisplatin, HMR 1275/Flavopiridol, CP-358 (774)/EGFR, CP-609 (754)/RAS oncogene inhibitor, BMS-182751/oral platinum, UFT^{™}(Tegafur/Uracil), ERGAMISOL^{®}/Levamisole, Eniluracil/776C85/5FU enhancer, Campto/Levamisole, CAMPTOSAR^{®}/Irinotecan, Tumodex/Ralitrexed, LEUSTATIN^{®}/Cladribine, Paxex/Paclitaxel, DOXIL^{®}/liposomal doxorubicin, Caelyx/liposomal doxorubicin, FLUDARA^{®}/Fludarabine, Pharmarubicin/Epirubicin, DEPOCYT^{®}, ZD1839, LU 79553/Bis-Naphtalimide, LU 103793/Dolastain, Caetyx/liposomal doxorubicin, GEMZAR^{®}/Gemcitabine, ZD 0473/ANORMED^{®}, YM 116, Iodine seeds, CDK4 and CDK2 inhibitors, PARP inhibitors, D4809/Dexifosamide, Ifes/MESNEX^{®}/Ifosamide, VUMON^{®})/Teniposide, PARAPLATIN^{®}/Carboplatin, Plantinol/cisplatin, Vepeside/Etoposide, ZD 9331, TAXOTERE^{®}/Docetaxel, prodrug of guanine arabinoside, Taxane Analog, nitrosoureas, alkylating agents such as melphelan and cyclophosphamide, Aminoglutethimide, Asparaginase, Busulfan, Carboplatin, Chlorombucil, Cytarabine HCl, Dactinomycin, Daunorubicin HCl, Estramustine phosphate sodium, Etoposide (VP 16-213), Floxuridine, Fluorouracil (5-FU), Flutamide, Hydroxyurea (hydroxycarbamide), Ifosfamide, Interferon Alfa-2a, Alfa-2b, Leuprolide acetate (LHRH-releasing factor analogue), Lomustine (CCNU), Mechlorethamine HCl (nitrogen mustard), Mercaptopurine, Mesna, Mitotanc (o.p'-DDD), Mitoxantrone HCl, Octreotide, Plicamycin, Procarbazine HCl, Streptozocin, Tamoxifen citrate, Thioguanine, Thiotcpa, Vinblastine sulfate, Amsacrine (m-AMSA), Azacitidine, Erthropoietin, Hexamethylmelamine (HMM), Interleukin 2, Mitoguazone (methyl-GAG; methyl glyoxal bis-guanylhydrazone; MGBG), Pentostatin (2'deoxycoformycin), Semustine (methyl-CCNU), Teniposide (VM-26) and Vindesine sulfate. Preferred monocloncal antibodies include, but are not limited to, PANOREX^{®} (Glaxo-Welicome), RITUXAN^{®} (IDEC/Genentech/Hoffman la Roche), MYLOTARG^{®} (Wyeth), CAMPATH^{®} (Millennium), ZEVALIN^{®} (IDEC and Schering AG), BEXXAR^{®} (Corixa/GSK), ERBITUX^{®} (Imclone/BMS), AVASTIN^{®} (Genentech) HERCEPTIN^{®} (Genentech/Hoffman la Roche), TARCEVA^{®}(OSI Pharmaceuticals/Genentech).

The following examples are intended to further illustrate certain preferred embodiments of the invention and are not intended to limit the scope of the invention in any way.

### Example 1: Synthesis of immunomers

Chemical entities according to the invention were synthesized on a 1 µmol to 0.1 mM scale using an automated DNA synthesizer (OligoPilot II, AKTA, (Amersham) and/or Expedite 8909 (Applied Biosystem)), following the linear synthesis or parallel synthesis procedures outlined in Figures 1 and 2.

5'-DMT dA, dG, dC and T phosphoramidites were purchased from Proligo (Boulder, CO). 5'-DMT 7-deaza-dG and araG phosphoramidites were obtained from Chemgenes (Wilmington, MA). DiDMT-glycerol linker solid support was obtained from Chemgenes. 1-(2'-deoxy-β-D-ribofuranosyl)-2-oxo-7-deaza-8-methyl-purine amidite was obtained from Glen Research (Sterling, VA), 2'-O-methylribonuncleoside amidites were obtained from Promega (Obispo, CA). All compounds according to the invention were phosphorothioate backbone modified.

All nucleoside phosphoramidites were characterized by ³¹P and ¹H NMR spectra. Modified nucleosides were incorporated at specific sites using normal coupling cycles recommended by the supplier. After synthesis, compounds were deprotected using concentrated ammonium hydroxide and purified by reverse phase HPLC, detritylation, followed by dialysis. Purified compounds as sodium salt form were lyophilized prior to use. Purity was tested by CGE and MALDI-TOF MS. Endotoxin levels were determined by LAL test and were below 1.0 EU/mg.

### Example 2: Intratumoral injection of TLR9 agonist compared to subcutaneous administration in an A20 lymphoma model

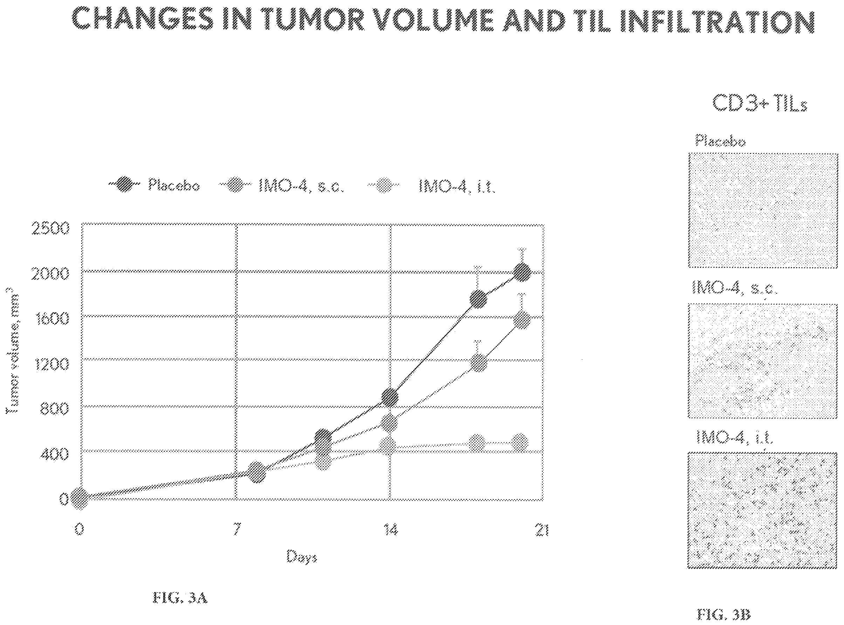

BALB/c mice (n=10) were implanted s.c with 3 x 10⁶ A20 cells on the right flank. Treatment was initiated on day 8 with either intratumoral (i.t.) or subcutaneous (s.c.) injection of 2.5 mg/kg IMO-4. IMO-4 was given on days 8, 10, 12 and 14. Samples from placebo (PBS) control and IMO-4 treated tumor-bearing mice were collected on day 21 after tumor implantation. As shown in Figures 3A and 3B, intratumoral IMO-4 induced potent antitumor activity and CD3+ TIL infiltration. Intratumoral IMO-4 also modulated tumor checkpoint expression compared to subcutaneous administration thereby sensitizing the tumor microenvironment for combination with one or more checkpoint inhibitors (data not shown).

### Example 3: Intratumoral injection of TLR9 agonist in an A20 lymphoma model

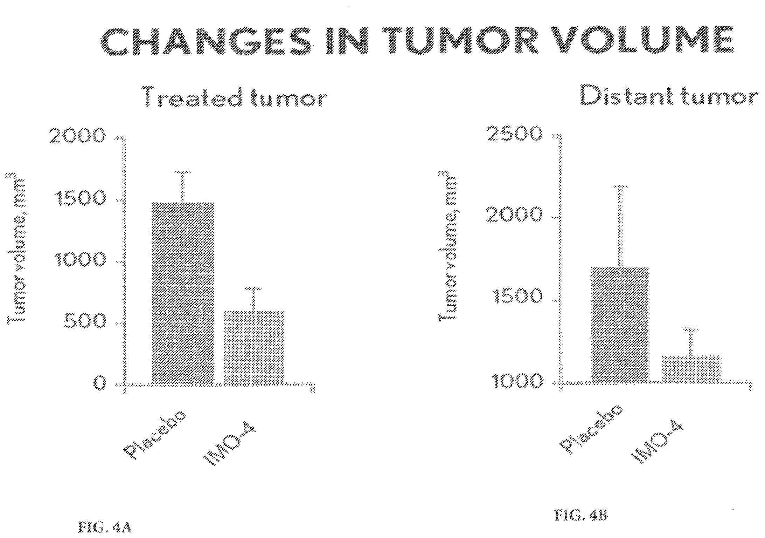

BALB/c mice (n=10) were implanted s.c with 3 × 10⁶ CT26 cells on the right and left flank. Treatment was initiated on day 8 with intratumoral injection in the left flank with 2.5 mg/kg IMO-4. IMO-4 was given on days 8, 10, 12, and 14. Samples from placebo (PBS) control and IMO-4 treated tumor-bearing mice were collected on day 21 after tumor implantation. As shown in Figure 4, intratumoral IMO-4 induced potent antitumor activity in both treated and distant tumor nodules. Intratumoral IMO-4 also modulated tumor checkpoint expression thereby sensitizing the tumor microenvironment for combination with one or more checkpoint inhibitors (data not shown).

### Example 4: Intratumoral injection of TLR9 agonist in a CT26 colon carcinoma model

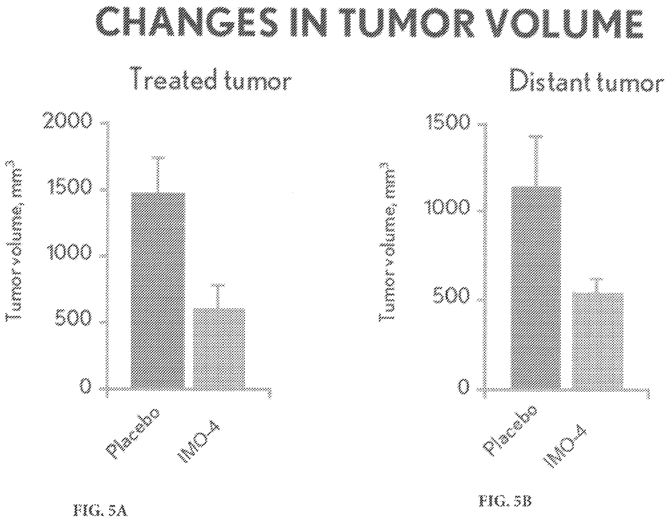

BALB/c mice (n=9) were implanted s.c with 2 x 10⁶ CT26 cells on the right and left flank. Treatment was initiated on day 7 with intratumoral injection in the left flank with 2.5 mg/kg IMO-4. IMO-4 was given on days 7, 9, 11, 13 and 15. Samples from placebo (PBS) control and IMO-4 treated tumor-bearing mice were collected on day 27 after tumor implantation. As shown in Figure 5, intratumoral IMO-4 induced potent antitumor activity in both treated and distant tumor nodules. Intratumoral IMO-4 also modulated tumor checkpoint expression thereby sensitizing the tumor microenvironment for combination with one or more checkpoint inhibitors (data not shown).

### Example 5: Intratumoral injection of TLR9 agonist in a B16 melanoma model

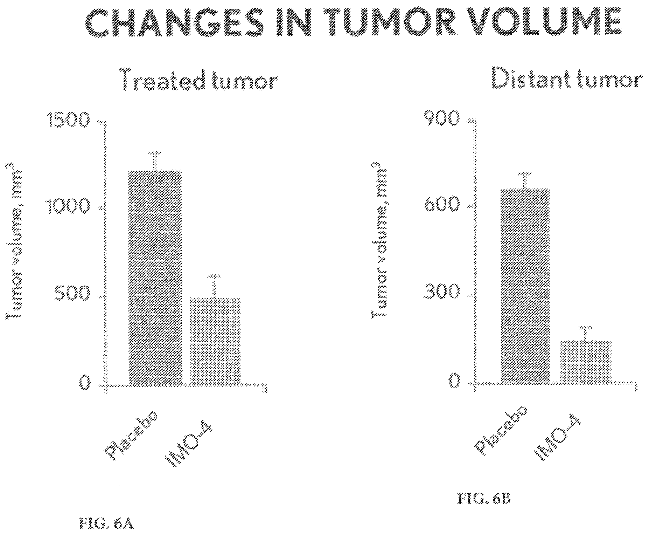

BALB/c mice (n=9) were implanted s.c with 1 × 10⁶ B16 cells on the right and left flank. Treatment was initiated on day 7 with intratumoral injection in the left flank with of 2.5 mg/kg IMO-4. IMO-4 was given on days 7, 9, 11, 13 and 15. Samples from placebo (PBS) control and IMO-4 treated tumor-bearing mice were collected on day 22 after tumor implantation. As shown in Figure 6, intratumoral IMO-4 induced potent antitumor activity in both treated and distant tumor nodules. Intratumoral IMO-4 also modulated tumor checkpoint expression thereby sensitizing the tumor microenvironment for combination with one or more checkpoint inhibitors (data not shown).

### Example 6: TLR9 agonist and checkpoint inhibitor combination therapy on treated tumors and lung metastases

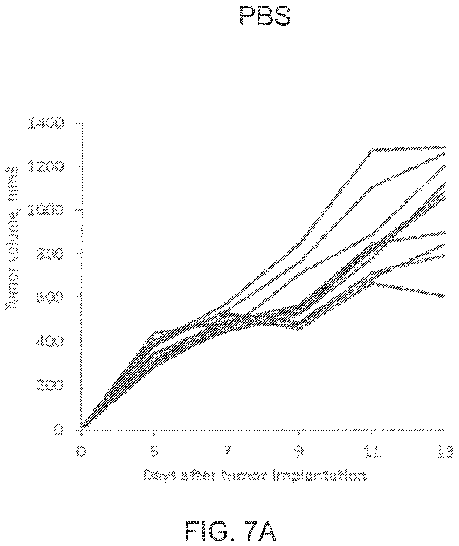

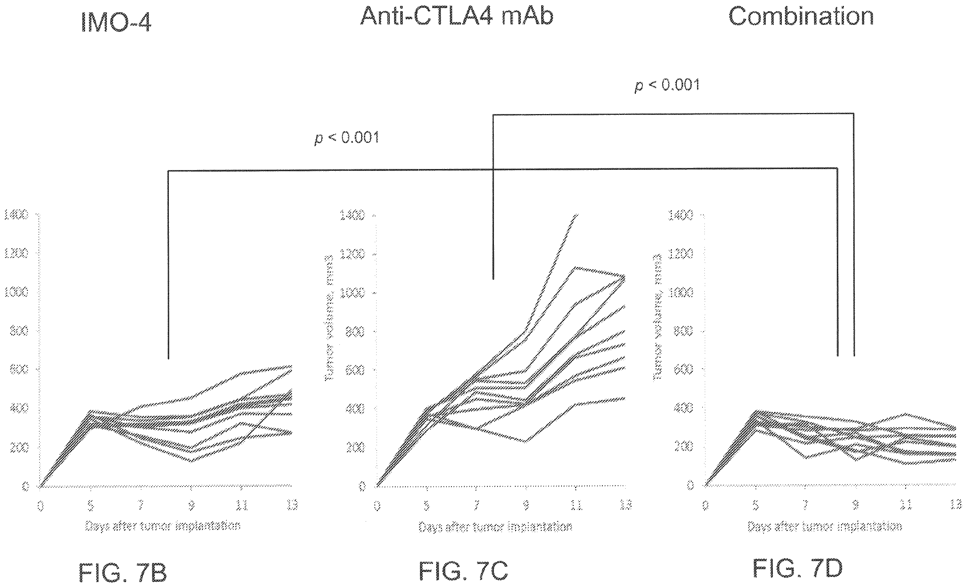

BALB/c mice were implanted s.c with 2 × 10⁷ CT26 cells on right flank. The mice were than i.v injected with 3 × 10⁶ CT26 cells to establish lung metastases. Treatment was initiated on day 5. 2.5 mg/kg IMO-4 was administered intratumorally into CT26 solid tumors on the right flank and 10 mg/kg anti-CTLA-4 mAb was administered by interperitoneal (i.p.) injection. IMO-4 and anti-CTLA4 mAb were given either alone or co-administered on days 5, 6, 8 and 9. Lungs and T cells from spleens of PBS control, IMO-4, anti-CTLA-4 mAb or IMO-4 and anti-CTLA-4 mAb treated tumor-bearing mice were collected. Figures 7 through 9 show the effects of IMO-4 and anti-CTLA-4 mAb on directly treated tumors and systemic lung metastasis.

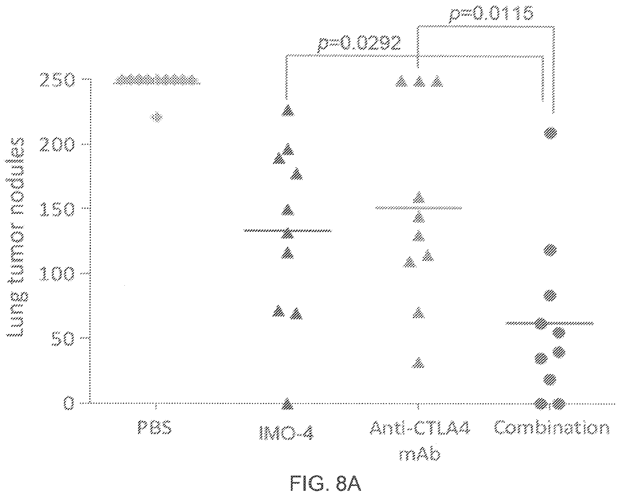

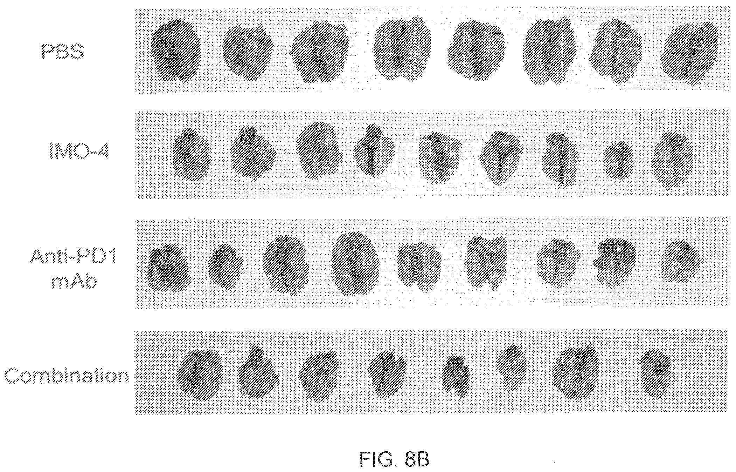

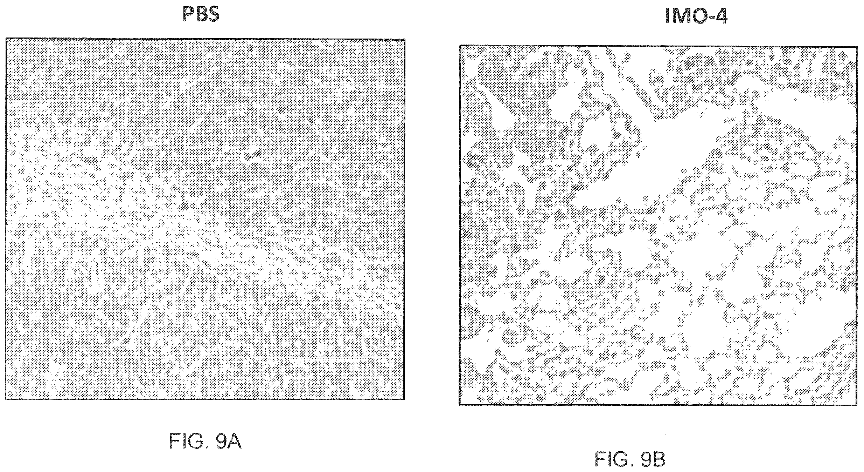

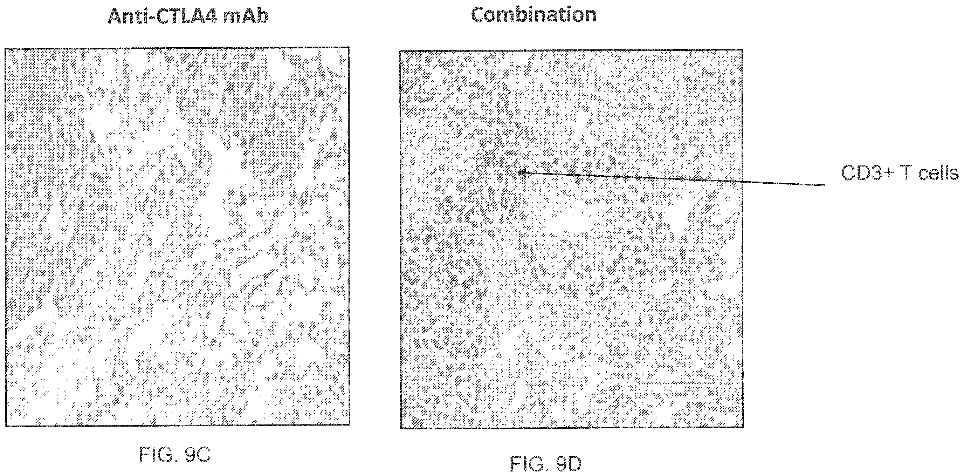

As shown in Figures 7 and 8, IMO-4 and anti-CTLA4 mAb combination therapy resulted in improved tumor growth inhibition versus IMO-4 or anti-CTLA4 mAb alone. As shown in Figure 9, the cytotoxic T cells against β-gal presented in the systemic lung metastasis sites were dramatically increased (p<0.01) compared to either monotherapy alone.

### Example 7: TLR9 agonist and checkpoint inhibitor combination therapy on treated and distant tumors

BALB/c mice (n=8 per group) were implanted s.c with 1 × 10⁷ murine colon carcinoma CT26 cells in right flank (Tumor 1) and left flank (Tumor 2). Treatment was initiated on day 7 when tumor volume on reaches 200 to 300 mm³. 2.5 mg/kg IMO-4 (50 µg in 100 µl PBS) was i.t injected at right tumor nodules and anti-PD-1 mAb (10 mg/kg, 200 µg/mouse) was administered by i.p injection either alone or co-administered on days 7, 8, 11 and 12 for total 4 times. Tumor nodules were collected at day 14.

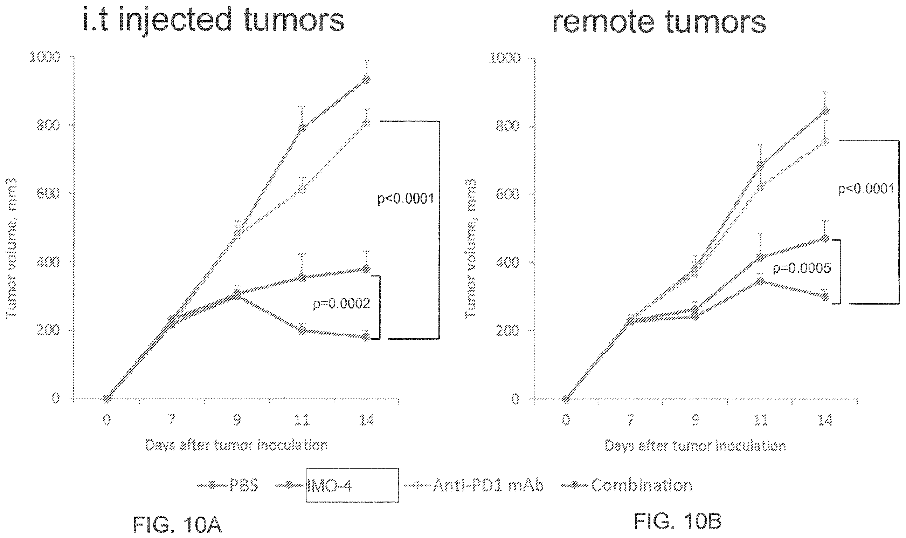

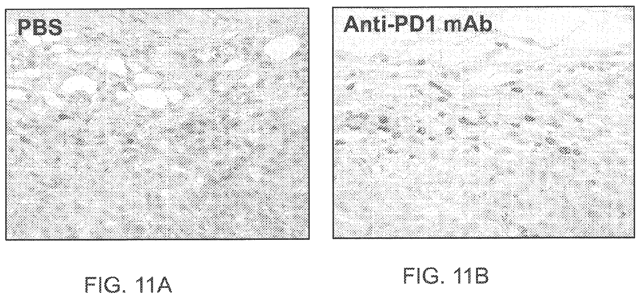

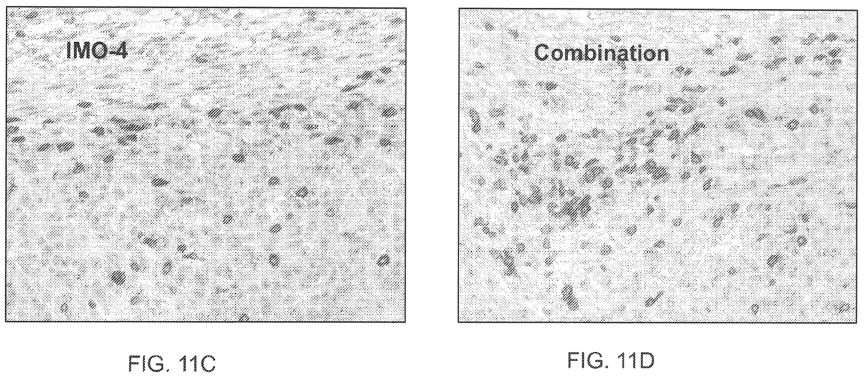

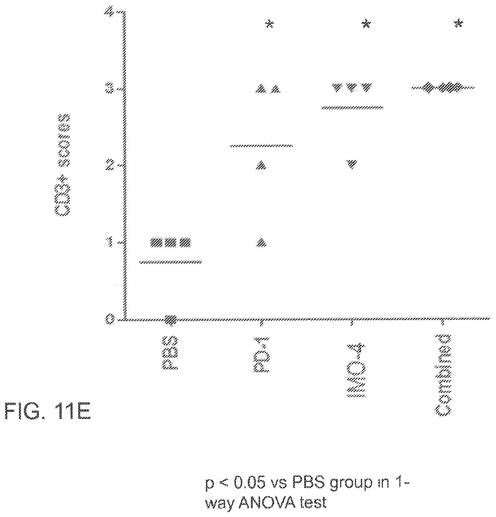

As shown in Figure 10, intratumoral injections of IMO-4 plus anti-PD-1 mAb on a single tumor lead to potent antitumor effects to both local (Fig. 10A) and distant tumors (Fig. 10B). Figure 11 demonstrates that IMO-4 increases T lymphocyte infiltration into tumor tissues. While few CD3+ cells present in the tumor tissue bordering normal tissue from PBS (vehicle) injected mice, a large number of CD+3 cells are presented in the tumor tissue from mice treated with IMO-4 or anti-PD-1 mAb. However, most abundant CD3+ cells are present in tumors from mice receiving combined treatment of IMO-4 and CTLA-4 mAb.

### Example 8: TLR9 agonist and checkpoint inhibitor combination therapy on treated tumors and systemic lung metastases

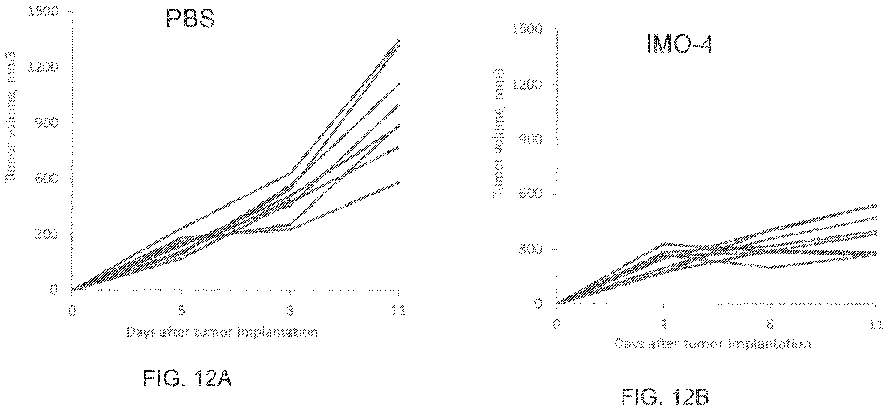

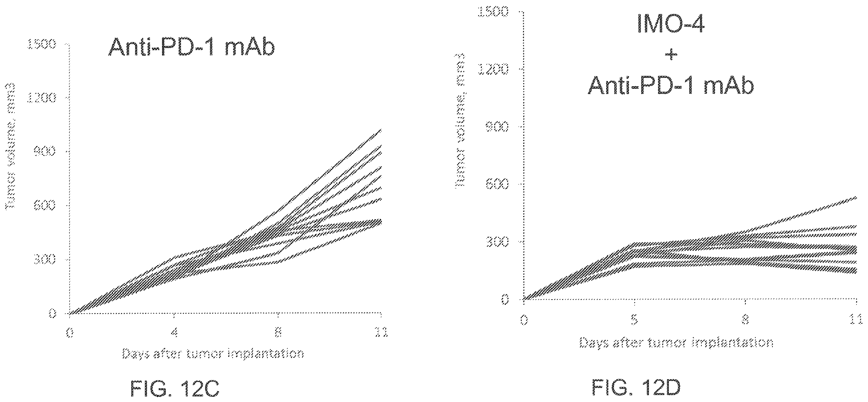

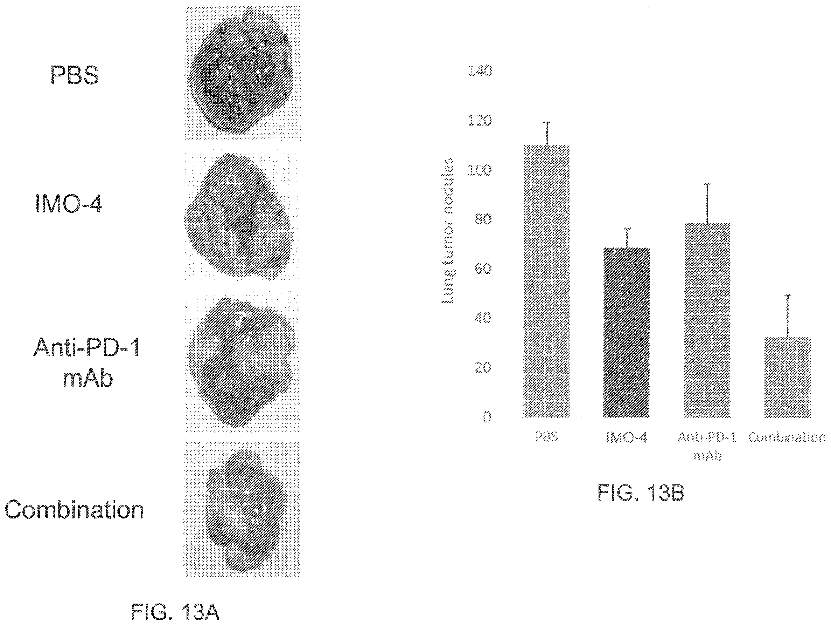

BALB/c mice were implanted s.c with 1 × 10⁷ B16.F10 cells on right flank. The mice were than i.v injected with 2 × 10⁶ B16.F10 cells to establish lung metastases. Treatment was initiated on day 5. 5 mg/kg IMO-4 was administered intratumorally into B16 solid tumors on the right flank and 15 mg/kg anti-PD-1 mAb was administered by interperitoneal (i.p.) injection. IMO-4 and anti-PD-1 mAb were given either alone or co-administered on days 5, 6, 7, 8, and 9. Samples from control, IMO-4, anti-PD-1 mAb or IMO-4 and anti-PD-1 mAb treated tumor-bearing mice were collected. Figures 12 and 13 show the effects of IMO-4 and anti-PD-1 mAb on directly treated tumors and systemic lung metastasis.

### Example 9: TLR9 agonist and checkpoint inhibitor combination therapy on treated tumors and systemic lung metastases

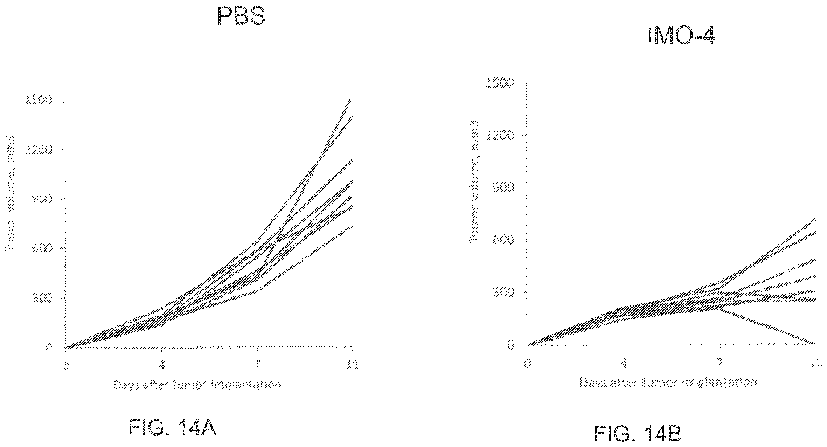

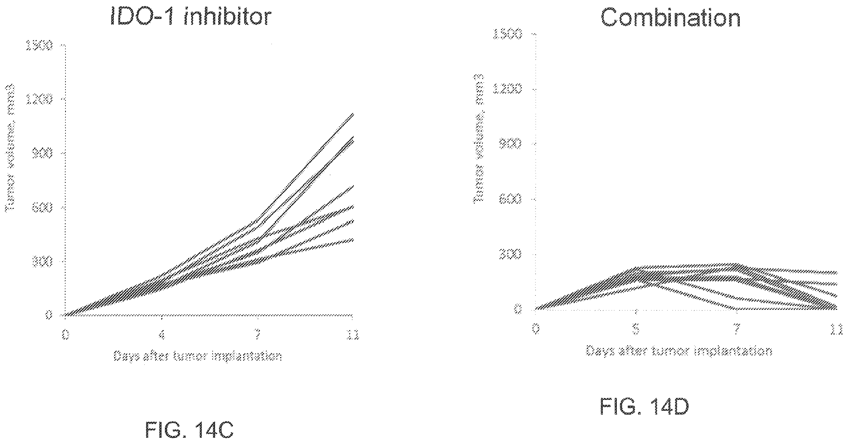

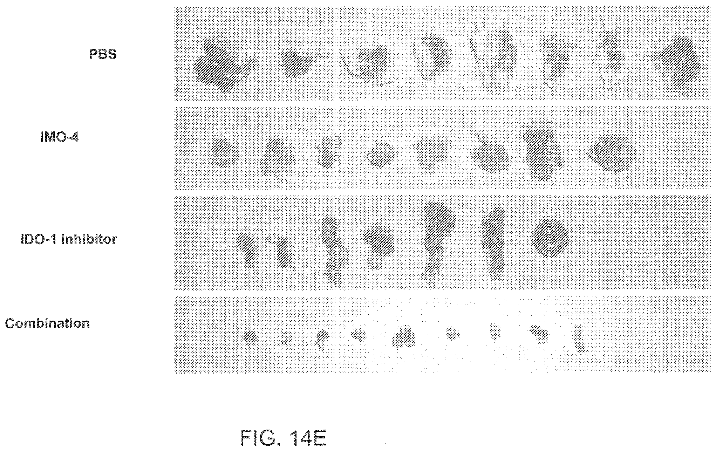

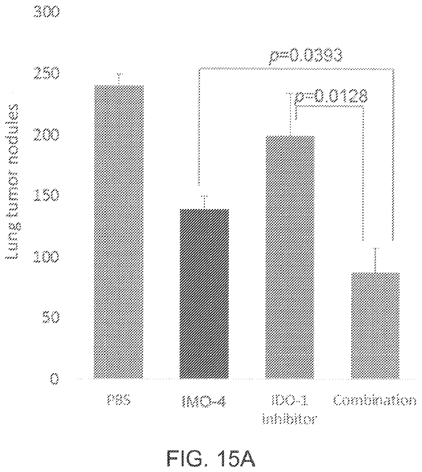

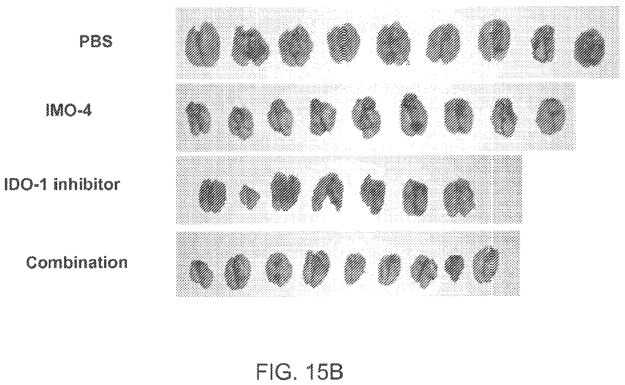

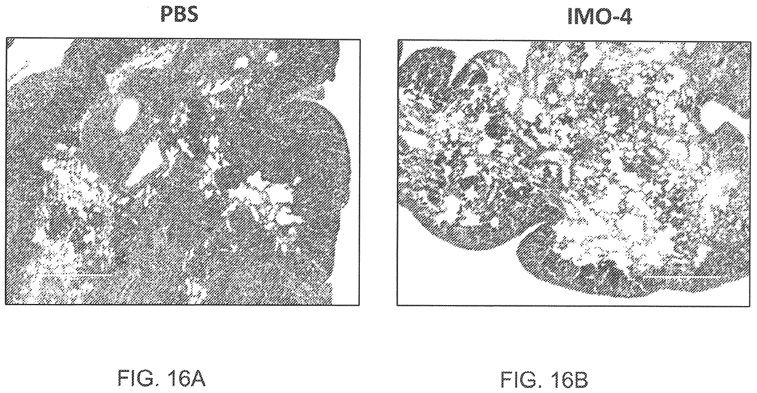

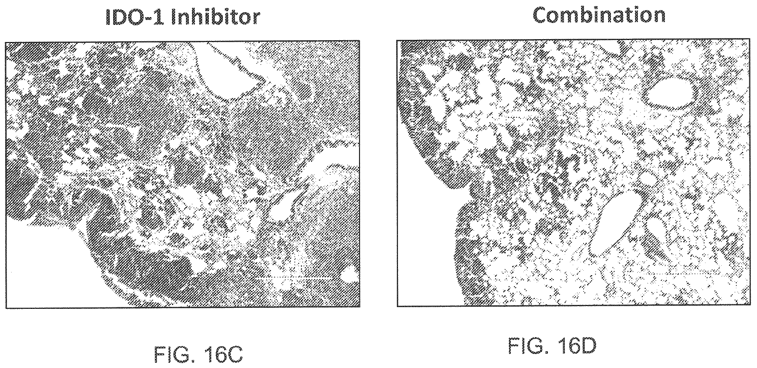

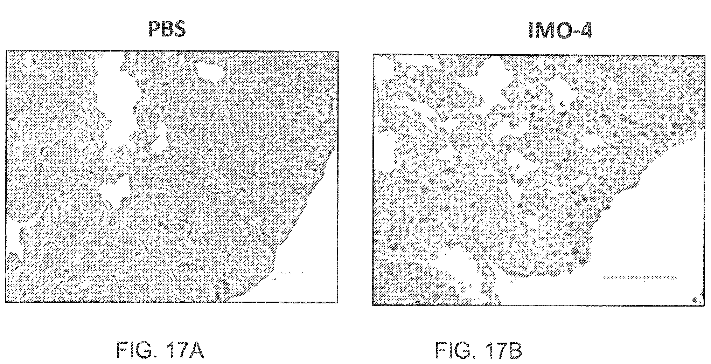

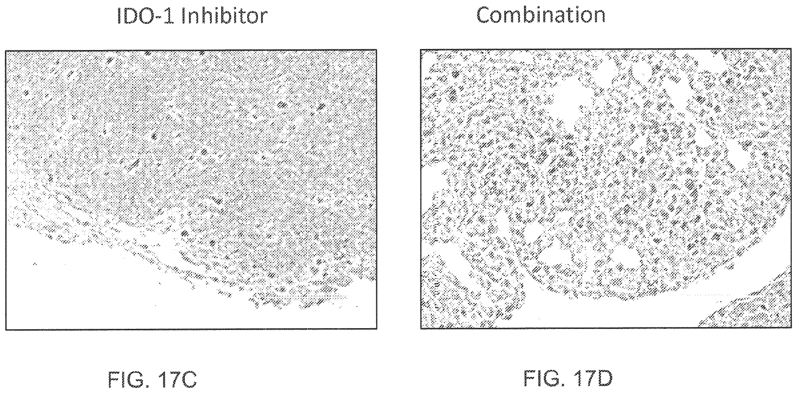

BALB/c mice were implanted s.c with 1 × 10⁷ CT26 cells on right flank. The mice were than i.v injected with 3 × 10⁶ CT26 cells to establish lung metastases. Treatment was initiated on day 4. 2.5 mg/kg IMO-4 was administered intratumorally into solid tumors on the right flank and 75 mg/kg anti-IDO1 inhibitor was administered orally (p.o.). IMO-4 and anti-IDOl inhibitor were given either alone or co-administered on days 4, 5, 7, and 8. Anti-IDOl was administered twice. Samples from control, IMO-4, anti-IDOl inhibitor or IMO-4 and anti-IDOl inhibitor treated tumor-bearing mice were collected. Figures 14 through 17 show the effects of IMO-4 and anti-IDO1 inhibitor on directly treated tumors and systemic lung metastasis.

### EQUIVALENTS

While the foregoing invention has been described in some detail for purposes of clarity and understanding, it will be appreciated by one skilled in the art from a reading of this disclosure that various changes in form and detail can be made without departing from the true scope of the invention and appended claims.

### ITEMS

Disclosed items:
1. A method for treating cancer in a patient, the method comprising the co-administration of one or more TLR9 agonists and one or more checkpoint inhibitors, wherein the one or more TLR9 agonists is administered intratumorally.
2. The method according to item 1, wherein the one or more TLR9 agonists and the one or more checkpoint inhibitors are each administered in a pharmaceutically effective amount.
3. The method according to item 2, wherein the one or more TLR9 agonist is administered prior to the patient being administered the one or more checkpoint inhibitor.
4. The method according to item 1, wherein the TLR9 agonist is an immunomer.
5. The method according to item 1, wherein the one or more checkpoint inhibitor are targeted to an immune checkpoint selected from the group consisting of CTLA4, PD-1, PD-L1, LAG3, B7-H3, B7-H4, KIR, OX40, IgG, IDO-1, IDO-2, CEACAM1, TNFRSF4, BTLA, OX40L, and TIM3 or combinations thereof.
6. The method according to item 5, wherein the one or more checkpoint inhibitor are targeted to an immune checkpoint selected from the group consisting of CTLA-4, IDO-1, PD-L1, and PD-1 or combinations thereof.
7. The method according to item 4, wherein the immunomer is selected from the group consisting of 5'-TCTGACG₁TTCT-X-TCTTG₁CAGTCT-5', 5'-TCTGTCG₁TTCT-X-TCTTG₁CTGTCT-5',5-TCG₁TCG₁TTCTG-X-GTCTTG₁CTG₁CT-5', 5-TCG₁AACG₁TTCG₁-X-G₁CTTG₁CAAG₁CT-5', 5-CTGTCoG₂TTCTC-X-CTCTTG₂oCTGTC-5', 5-CTGTCG₂TTCTCo-X-oCTCTTG₂CTGTC-5', 5'-TCG₁AACG₁TTCG₁-X-TCTTG₂CTGTCT-5', 5-TCG₁AACG₁TTCG₁-Y-GACAG₁CTGTCT-5', 5-CAGTCG₂TTCAG-X-GACTTG₂CTGAC-5', 5-CAGTCG₁TTCAG-X-GACTTG₁CTGAC-5', 5'-TCG₁AACG₁TTCoG-Z-GoCTTG1CAAG₁CT-5', 5'-TCG₁AACG₁TTCG₁-Y₂-TCTTG₁CTGTCTTG₁CT-5', 5'-TCG₁AACG₁TTCG₁-Y₂-TCTTG₁CTGUCT-5', 5'-TCG₁AACG1ToTCoG-m-GoCToTG₁CAAG₁CT-5', 5'-TCG₁AACG₁TTCoG-Y₃-GACTTG₂CTGAC-5', 5'-TCG₁AACG₁TTCG₁-Y₄-TGTTG₁CTGTCTTG₁CT-5', 5'-TCG₂TCG₂TTU₁Y-M-YU₁TTG₂CTG₂CT-5', and 5'-CAGTCG₂TTCAG-Y₃-TCTTG₁CTGTCT-5', wherein G₁ is 2'-deoxy-7-deazaguanosine; G₂ is 2'-deoxy-arabinoguanosine; G/C/U is 2'-O-methylribonucleotides; U₁ is 2'-deoxy-U; o is phosphodiester linkage; X is a glycerol linker; Y is C3-linker; m is cis,trans-1,3,5-cyclohexanetriol linker; Y₂ is 1,3-propanediol linker; Y₃ is 1,4-butanediol linker; Y₄ is 1,5-pentandiol linker; Z is 1,3,5-pentanetriol linker; M is cis,cis-1,3,5-cyelohexanetriol linker.
8. The method according to item 7, wherein the immunomer comprises the sequence 5-TCG₁AACG₁TTCG₁-X-G₁CTTG₁CAAG₁CT-5, wherein G₁ is 2'-deoxy-7-deazaguanosine and X is a glycerol linker.
9. The method according to item 1, wherein cancer is selected from the group consisting of non-Hodgkin's lymphoma, B cell lymphoma, B cell leukemia, T cell lymphoma, T cell leukemia, acute lymphoid leukemia, chronic lymphoid leukemia, Burkitt lymphoma, Hodgkin's lymphoma, hairy cell leukemia, acute myeloid leukemia, chronic myeloid leukemia, multiple myeloma, glioma, Waldenstrom's macroglobulinemia, carcinoma, melanoma, sarcoma, glioma, skin cancer, oral cavity cancer, gastrointestinal tract cancer, colon cancer, stomach cancer, pulmonary tract cancer, lung cancer, breast cancer, ovarian cancer, prostate cancer, uterine cancer, endometrial cancer, cervical cancer, urinary bladder cancer, pancreatic cancer, bone cancer, liver cancer, gall bladder cancer, kidney cancer, and testicular cancer.
10. The method according to item 9, wherein the cancer is melanoma.
11. A method for treating cancer in a patient, the method comprising the co-administration of one or more TLR9 agonists, a checkpoint inhibitor of IDO1, and one or more additional checkpoint inhibitors, wherein the one or more TLR9 agonists is administered intratumorally.
12. The method according to item 11, wherein the one or more TLR9 agonists and the one or more checkpoint inhibitors are each administered in a pharmaceutically effective amount.
13. The method according to item 12, wherein the one or more TLR9 agonist is administered prior to the patient being administered the checkpoint inhibitor of IDO1 and the one or more additional checkpoint inhibitors.
14. The method according to item 11, wherein the TLR9 agonist is an immunomer.
15. The method according to item 11, wherein the one or more additional checkpoint inhibitors are targeted to an immune checkpoint selected from the group consisting of CTLA4, PD-1, PD-L1, LAG3, B7-H3, B7-H4, KIR, OX40, IgG, IDO-2, CEACAM1, TNFRSF4, BTLA, OX40L, and TIM3 or combinations thereof.
16. The method according to item 15, wherein the one or more checkpoint inhibitor are targeted to an immune checkpoint selected from the group consisting of CTLA-4, IDO-1, PD-L1, and PD-1 or combinations thereof.
17. The method according to item 14, wherein the immunomer is selected from the group consisting of 5'-TCTGACG₁TTCT-X-TCTTG₁CAGTCT-5', 5'-TCTGTCG₁TTCT-X-TCTTG₁CTGTCT-5',5-TCG₁TCG₁TTCTG-X-GTCTTG₁CTG₁CT-5', 5-TCG₁AACG₁TTCG₁-X-G₁CTTG₁CAAG₁CT-5', 5-CTGTCoG₂TTCTC-X-CTCTTG₂oCTGTC-5', 5-CTGTCG₂TTCTCo-X-oCTCTTG₂CTGTC-5', 5'-TCG₁AACG₁TTCG₁-X-TCTTG₂CTGTCT-5', 5-TCG₁AACG₁TTCG₁-Y-GACAG₁CTGTCT-5',5-CAGTCG₂TTCAG-X-GACTTG₂CTGAC-5', 5-CAGTCG₁TTCAG-X-GACTTG₁CTGAC-5', 5'-TCG₁AACG₁TTCoG-Z-GoCTTG₁CAAG₁CT-5',5'-TCG₁AACG₁TTCG₁-Y₂-TCTTG₁CTGTCTTG₁CT-5',5'-TCG₁AACG₁TTCG₁-Y₂-TCTTG₁CTGUCT-5', 5'-TCG₁AACG₁ToTCoG-m-GoCToTG₁CAAG₁CT-5', 5'-TCG₁AACG₁TTCoG-Y₃-GACTTG₂CTGAC-5', 5'-TCG₁AACG₁TTCG₁-Y₄-TGTTG₁CTGTCTTG₁CT-5', 5'-TCG₂TCG₂TTUᵢY-M-YU₁TTG₂CTG₂CT-5', and 5'-CAGTCG₂TTCAG-Y₃-TCTTG₁CTGTCT-5', wherein G₁ is 2'-deoxy-7-deazaguanosine; G₂ is 2'-deoxy-arabinoguanosine; G/C/U is 2'-O-methylribonucleotides; U₁ is 2'-deoxy-U; o is phosphodiester linkage; X is a glycerol linker; Y is C3-linker; m is cis,trans-1,3,5-cyclohexanetriol linker; Y₂ is 1,3-propanediol linker; Y₃ is 1,4-butanediol linker; Y₄ is 1,5-pentandiol linker; Z is 1,3,5-pentanetriol linker; M is cis,cis-1,3,5-cyclohexanetriol linker.
18. The method according to item 17, wherein the immunomer comprises the sequence 5-TCG₁AACG₁TTCG₁-X-G₁CTTG₁CAAG₁CT-5, wherein G₁ is 2'-deoxy-7-deazaguanosine and X is a glycerol linker.
19. The method according to item 11, wherein cancer is selected from the group consisting of non-Hodgkin's lymphoma, B cell lymphoma, B cell leukemia, T cell lymphoma, '1' cell leukemia, acute lymphoid leukemia, chronic lymphoid leukemia, Burkitt lymphoma, Hodgkin's lymphoma, hairy cell leukemia, acute myeloid leukemia, chronic myeloid leukemia, multiple myeloma, glioma, Waldenstrom's macroglobulinemia, carcinoma, melanoma, sarcoma, glioma, skin cancer, oral cavity cancer, gastrointestinal tract cancer, colon cancer, stomach cancer, pulmonary tract cancer, lung cancer, breast cancer, ovarian cancer, prostate cancer, uterine cancer, endometrial cancer, cervical cancer, urinary bladder cancer, pancreatic cancer, bone cancer, liver cancer, gall bladder cancer, kidney cancer, and testicular cancer.
20. The method according to item 1, wherein the treatment of a cancer further comprises administering an additional anticancer agent.
21. The method according to item 20, wherein the additional anticancer agent is selected from among a chemotherapeutic agent or radiation therapy.
22. The method according to item 21, wherein the chemotherapeutic agent is selected from among chlorambucil, ifosfamide, doxorubicin, mesalazine, thalidomide, lenalidomide, temsirolimus, everolimus, fludarabine, fostamatinib, paclitaxel, docetaxel, ofatumumab, rituximab, dexamethasone, prednisone, CAL-101, ibritumomab, tositumomab, bortezomib, pentostatin, endostatin, or a combination thereof.
23. The method according to item 11, wherein the treatment of a cancer further comprises administering an additional anticancer agent.
24. The method according to item 23, wherein the additional anticancer agent is selected from among a chemotherapeutic agent or radiation therapy.
25. The method according to item 24, wherein the chemotherapeutic agent is selected from among chlorambucil, ifosfamide, doxorubicin, mesalazine, thalidomide, lenalidomide, temsirolimus, everolimus, fludarabine, fostamatinib, paclitaxel, docetaxel, ofatumumab, rituximab, dexamethasone, prednisone, CAL-101, ibritumomab, tositumomab, bortezomib, pentostatin, endostatin, or a combination thereof.

## Claims

1. A composition for use in treating a solid tumor in a patient with metastatic cancer, the composition comprising:
a TLR9 agonist having the structure 5'-TCG1AACG1TTCG1-X-G1CTTG1CAAG1CT-5' (5'-SEQ ID NO:4-X-SEQ ID NO:4-5'), wherein G1 is 2'-deoxy-7-deazaguanosine and X is a glycerol linker; and
wherein the composition is to be co-administered with more than one inhibitor targeting immune checkpoints selected from the group consisting of CTLA-4, PD-1, PD-L1, IDO-1, LAG3, B7-H3, B7-H4, KIR, OX40, IgG, IDO-2, CEACAM1, TNFRSF4, BTLA, OX40L, and TIM3; and
wherein the TLR9 agonist is to be administered intratumorally to the solid tumor.

2. The composition for the use according to claim 1, wherein the TLR9 agonist and the more than one inhibitor targeting immune checkpoints selected from the group consisting of CTLA-4, PD-1, PD-L1, IDO-1, LAG3, B7-H3, B7-H4, KIR, OX40, IgG, IDO-2, CEACAM1, TNFRSF4, BTLA, OX40L, and TIM3, are each to be administered in a pharmaceutically effective amount.

3. The composition for the use according to claim 2, wherein the TLR9 agonist is to be administered prior to the patient being administered the more than one inhibitor targeting immune checkpoints selected from the group consisting of CTLA-4, PD-1, PD-L1, IDO-1, LAG3, B7-H3, B7-H4, KIR, OX40, IgG, IDO-2, CEACAM1, TNFRSF4, BTLA, OX4OL, and TIM3.

4. The composition for the use according to claim 2, wherein the TLR9 agonist is to be administered to the patient concurrently with the more than one inhibitor targeting immune checkpoints selected from the group consisting of CTLA-4, PD-1, PD-L1, IDO-1, LAG3, B7-H3, B7-H4, KIR, OX40, IgG, IDO-2, CEACAM1, TNFRSF4, BTLA, OX40L, and TIM3.

5. The composition for the use according to claim 1, wherein the more than one inhibitor targeting immune checkpoints are targeted to immune checkpoints selected from the group consisting of CTLA-4, IDO-1, PD-L1, and PD-1 or combinations thereof.

6. The composition for the use according to claim 5, wherein the more than one inhibitor of CTLA-4, PD-1, PD-L1, and/or IDO-1 include an inhibitor of CTLA-4 that is an antibody against CTLA-4.

7. The composition for the use according to claim 1, wherein the metastatic cancer is selected from the group consisting of non-Hodgkin's lymphoma, B cell lymphoma, B cell leukemia, T cell lymphoma, T cell leukemia, acute lymphoid leukemia, chronic lymphoid leukemia, Burkitt lymphoma, Hodgkin's lymphoma, hairy cell leukemia, acute myeloid leukemia, chronic myeloid leukemia, multiple myeloma, glioma, Waldenstrom's macroglobulinemia, carcinoma, melanoma, sarcoma, glioma, skin cancer, oral cavity cancer, gastrointestinal tract cancer, colon cancer, stomach cancer, pulmonary tract cancer, lung cancer, breast cancer, ovarian cancer, prostate cancer, uterine cancer, endometrial cancer, cervical cancer, urinary bladder cancer, pancreatic cancer, bone cancer, liver cancer, gall bladder cancer, kidney cancer, and testicular cancer.

8. The composition for the use according to claim 7, wherein the metastatic cancer is melanoma.

9. The composition for the use according to claim 5, wherein the more than one inhibitor of CTLA-4, PD-1, PD-L1, and/or IDO-1 include an inhibitor of IDO-1.

10. The composition for the use according to claim 9, wherein the TLR9 agonist is to be administered prior to the patient being administered the checkpoint inhibitor of IDO-1.

11. The composition for the use according to claim 1, wherein an additional anticancer agent is to be administered, wherein the additional anticancer agent is selected from among a chemotherapeutic agent or radiation therapy.

12. The composition for the use according to claim 1, wherein the more than one inhibitor of CTLA-4, PD-1, PD-L1, IDO-1, LAG3, B7-H3, B7-H4, KIR, OX40, IgG, IDO-2, CEACAM1, TNFRSF4, BTLA, OX40L, and TIM3 are to be administered parenterally or intratumorally.

13. The composition for the use according to claim 1, wherein the more than one inhibitor targeting immune checkpoints are targeted to immune checkpoints selected from the group consisting of CTLA-4, PD-1, PD-L1, IDO-1, LAG3, OX40, and TIM3.
